(19) Europäisches Patentamt
European Patent Office
Office européen des brevets

(11) EP 2 975 049 A1

(12) # EUROPEAN PATENT APPLICATION

(43) Date of publication:
**20.01.2016 Bulletin 2016/03**

(51) Int Cl.:
***C07K 14/705*** *(2006.01)*

(21) Application number: **14177633.6**

(22) Date of filing: **18.07.2014**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicants:
- **Centre National de la Recherche Scientifique (CNRS)**
  **75016 Paris (FR)**
- **UNIVERSITE DE MONTPELLIER**
  **34090 Montpellier (FR)**
- **Institut National de la Santé et de la Recherche Médicale (INSERM)**
  **75654 Paris Cedex 13 (FR)**

(72) Inventors:
- **Charnet, Pierre**
  **34270 Saint-Mathieu-de-Tréviers (FR)**
- **Cens, Thierry**
  **34920 Le Crès (FR)**
- **Rousset, Matthieu**
  **34000 Montpellier (FR)**

(74) Representative: **Icosa**
**83 avenue Denfert-Rochereau**
**75014 Paris (FR)**

(54) **GABA channel subunits of pollinator insects and their uses thereof**

(57) The present invention relates to the cloning of the GABA-gated ion channel receptor subunits in pollinator insect such as *Apis mellifera* and their uses thereof.

EP 2 975 049 A1

## Description

### FIELD OF INVENTION

The present invention relates to the cloning of the γ-Aminobutyric acid-gated ion channel receptor subunits in a pollinator insect such as *Apis mellifera* and their uses thereof.

### BACKGROUND OF INVENTION

During the last decades, agriculture has changed to meet the increasing demand to produce food. There has been expansion of cultivated areas in monoculture and increases use of pesticides. The abusive use of pesticides is subjecting pollinator insects to stress as evidenced by a constant decrease in the density of these pollinator insects around agricultural fields in many parts of the world, thus causing economic losses. Pollinators are crucial for the pollination of agricultural crops and natural areas around the world.

Among these pollinator insects, some insects issued from the order of *hymenoptera* or bees are considered excellent pollinating insects in agro-ecosystems because they visit many flowers on the same day. Among these pollinator insects, Bumble bees for instance have already been red-listed and are in danger of extinction.

Another bee such as *Apis mellifera* or honey bee is interesting from an economic perspective because it provides products of great value, such as honey, propolis, royal jelly, wax, and apitoxin (bee venom).

The decline of the bee colonies and pollinator insects due to pesticides is not only marked by the increase of their bee mortality but also modifications related to their memory or social behavior within the hives (Suchail S et al 2001 Environ Toxicol Chem 20 (11):2482-6; Gels JA 2002 J Econ Entomol; 95(4):722-8). Some toxicity assays have already been developed by oral or topical application of a substance on the insect however these assays are not reproducible and are not adapted to the test of a large diversity of compounds (OECD guidelines for the testing of chemicals OECD214 - September 1998). Consequently there is a need to develop high throughput assays highly reproducible and *in vitro* to determine whether a compound is toxic for pollinator insects.

γ-Aminobutyric acid (GABA)-gated chloride channel receptors are abundant in CNS of both vertebrates and invertebrates, where their physiological role is to mediate fast inhibitory neurotransmission. Native neuronal ionotropic chloride-selective GABA receptors have been studied in honeybee (Barbara et al. 2005 J Comp Physiol A Neuroethol Sens Neural Behav Physiol 191: 823-836; Grünewald and Wersing 2008 J Comp Physiol A Neuroethol Sens Neural Behav Physiol 194: 329-340). Insect GABA-gated chloride channels are the main targets of several classes of insecticidal active compounds including fipronil, picrotoxin, and dieldrin (Bloomquist 2003 Arch Insect Biochem Physiol 54: 145-156; Raymond and Sattelle 2002 Nat Rev Drug Discov 1: 427-436). From a molecular perspective, these GABA receptors belong to the dicysteine-loop ("cys-loop") superfamily of ligand-gated ion channels (LGICs), including both cation-permeable and anion-permeable channels (Raymond and Sattelle 2002).

The present application indeed unravels the yet unknown function of nucleic acid sequences and protein sequences of GABA-gated ion channel from pollinator insects whose activity is modulated by some toxic compounds. Consequently, the present application discloses a method using the nucleic acid sequences GABA-gated ion channel subunits from pollinator insects to analyze the effect of compounds on GABA-gated ion channel activity.

### SUMMARY

One object of the present invention is a nucleic acid sequence of the Resistant to DieLdrin (RDL) subunit as set forth in SEQ ID NO: 1 or variant thereof consisting of a nucleic acid sequence of less than 3400 bp and more than 1350 bp and having at least 90% identity with SEQ ID NO: 1.

Another object of the present invention is a nucleic acid sequence of the γ-Aminobutyric acid (GABA) and glycine-like receptor of *Drosophila* (GRD) subunit as set forth in SEQ ID NO: 3 or variant thereof consisting of a nucleic acid sequence of less than 2190 bp and more than 1395 bp and having at least 90% identity with SEQ ID NO: 3.

Another object of the present invention is a nucleic acid sequence of the ligand-gated ion channel homologue 3 (LCCH3) subunit as set forth in SEQ ID NO: 5 or variant thereof consisting of a nucleic acid sequence of more than 1470 bp and less than 1950 bp and having at least 90% identity with SEQ ID NO: 5.

Another object of the present invention is a vector comprising at least one nucleic acid sequence cited above.

Another object of the present invention is a modified cell expressing at least one vector of the invention.

Another object of the present invention is a GABA-gated ion channel of a pollinator insect comprising a RDL subunit as set forth in SEQ ID NO: 1 or variant thereof consisting of a nucleic acid sequence of less than 3400 bp and more than 1350 bp and having at least 90% identity with SEQ ID NO: 1.

In one embodiment, the GABA-gated ion channel further comprises at least one other subunit of said channel, wherein said other subunit is GRD and/or LCCH3 having the sequences as described above.

In another embodiment, the GABA-gated ion channel of a pollinator insect comprises the GRD and LCCH3 subunits having the sequences as described above.
Another object of the present invention is a vector comprising the channel as described above.
Another object of the present invention is a modified cell expressing a channel as described above or a vector as described above.
In one embodiment, the modified cell is an oocyte of Xenopus.
Another object of the present invention is an *in vitro* method to determine the effect of a test compound on the modulation of activity of a GABA-gated ion channel of a pollinator insect comprising:

a. contacting modified cells with at least one test compound,
b. measuring the effect of said test compound on the channel activity, and comparing said effect to the effect without test compound or to the effect of a reference value, thereby determining a modulation of activity of said channel.

In one embodiment, the method is for determining the toxicity of a test compound on a pollinator insect.
Another object of the present invention is an *in vitro* method for screening compounds that modulate the GABA-gated ion channel activity of pollinator insects comprising:

a. contacting modified cells with at least one test compound,
b. measuring the effect of said compound on the GABA-gated ion channel activity, and

comparing said effect to the effect without test compound or to the effect of a reference value, thereby determining a modulation of activity of said GABA-gated ion channel.
Another object of the present invention is a kit comprising at least one vector as described above or a modified cell as described above and reagents.

**DEFINITIONS**

In the present invention, the following terms have the following meanings:

- **"Nucleic acid sequence"** refers to encompass nucleic acids having the sequences set forth below as well as variants thereof including for example fragments, deletions, insertions and substitutions that maintain the ability to encode the different subunits of the GABA-gated ion channel of pollinator insects.

- **"Functional channel", "functional expression"** refer to the synthesis and any necessary post-translational processing of a channel molecule and/or at least one of its regulatory subunit in a modified cell so that the channel or its regulatory subunit is inserted properly in the cell membrane and is capable of conducting ions in response to an exposure to appropriate pharmacological agents.

- **"Test compound"** refers to a phytosanitary product, a molecule, an organism or extract thereof eventually able to bind and/or modulate the GABA-gated ion channel activity of a pollinator insect.

- **"Derivative"** or **"analog"** of a compound refers broadly to the modification or substitution of one or more chemical moieties on a parent compound and may include functional derivatives, positional isomers, tautomers, zwitterions, enantiomers, diastereomers, racemates, isosteres or stereochemical mixtures thereof.

- **"Phytosanitary product"** refers to biological or chemical compounds used as insecticides, herbicides, fungicides, fertilizers, antibiotics, or any products used in agriculture, in wine-making, food storage, ship bottom, for animals or domestic purposes.

- **"Sub-lethal doses"** refer to a concentration of a potentially lethal test compound that is not high enough to cause death meaning a concentration under the median lethal dose ($LD_{50}$) but still able to trigger death by a mechanism which is not acute (immediate) death. At sub-lethal dose, the test compound may induce changes in biological mechanisms that include but are not limited to: colony level behavioral changes, individual level behavioral changes, memory changes, cellular mechanisms or molecular mechanisms changes. The determination of mortality or some of these biological mechanisms changes are well-known in the state of the art. Therefore, technics determining such changes or mortality can easily be determined by the skilled artisan.

- **"Minimal modulation doses"** refer to the lowest concentration of a test compound required to modulate the GABA-gated ion channel activity.

- **"Modulation", "modulating"** or **"modulator"** of GABA-gated ion channel activity refers to the three distinct states that can be modulated and in which a GABA-gated ion channel can be configured: closed, open and desensitized. The binding of 2 to 5 ligands to the channel makes the transition between a closed (or a resting state) to an open (activated) state. Depending on the subunits constituting the GABA-gated ion channel the exposure time or the dose of the agonist (and optionally the co-agonist) the GABA-gated ion channel transits to a desensitized state. It also refers to compounds including **"agonists", "antagonists", "positive allosteric modulators", "negative allosteric modulators", "non-competitive channel blockers", "open channel blockers"** that may affect the current flow within the GABA-gated ion channel by inducing modification(s) of conformation on said GABA-gated ion channel, subunits assembly or organization, modification of the targeting, trafficking of the channels to the plasma membrane, modification of the life time of the said GABA-gated ion channel to the plasma membrane, or blocking the channel gate or any modification of post-translational state like phosphorylating state of the said GABA-gated ion channel.

- **"Agonists"** refer to compounds that bind to the receptor site where GABA binds (is also referred as the "active" or "orthosteric" site) and activate it, resulting in increased channel conductance. Examples of agonists include but are not limited to: GABA, Gaboxadol, Ibotenic Acid, Muscimol, and Progabide.

- **"Antagonists"** refer to compounds that bind to the receptor site where GABA binds but do not activate it. Though they have no effect on their own, antagonists compete with GABA for binding and thereby inhibit its action, resulting in decreased channel conductance. Examples of antagonists include but are not limited to: Bicuculline, Gabazine.

- **"Positive allosteric modulators"** refer to compounds that bind to allosteric sites on the receptor complex causing increased efficiency of the main site and therefore an indirect increase in Cl- conductance. Examples of positive allosteric modulators include but are not limited to: Barbiturates, Benzodiazepines, Carisoprodol, thienodiazepines, Ethanol (Alcohol), Etomidate, Glutethimide, Kavalactones, Meprobamate, Methaqualone, Neuroactive Steroids, Niacin/Niacinamide, Propofol, Stiripentol, Theanine, Valerenic Acid, Volatile/Inhaled Anesthetics, Lanthanum.

- **"Negative allosteric modulators"** refer to compounds that bind to allosteric site on the receptor complex causing decreased efficiency of the main site and therefore an indirect decrease in channel conductance. Examples of negative allosteric modulators include but are not limited to: Flumazenil, Ro15-4513, Sarmazenil, Zinc.

- **"Open channel blockers"** refer to compounds that block the ion channel pore once it is open, and thus prolong ligand-receptor occupancy, slow activation kinetics and inhibit ion flux in a subunit configuration-dependent and sensitization-state dependent manner. Examples of open channel blockers include but are not limited to: isoflurane, sevoflurane.

- **"Non-competitive channel blockers"** refer to compounds that bind to or near the central pore of the receptor complex and directly block channel conductance through the ion channel. Examples of non-competitive channel blockers include but are not limited to: Cicutoxin, Oenanthotoxin, Pentylenetetrazol, Picrotoxin, Thujone, Lindane.

- **"Am"** or **"Amel"** preceding any gene's name refer to the pollinator insect *Apis mellifera*.

**DETAILED DESCRIPTION**

One object of the present invention is the isolated nucleic acid sequences or a variant thereof comprising at least one subunit of the γ-Aminobutyric acid (GABA)-gated ion channel receptor of a pollinator insect.

GABA-gated ion channel is also named ionotropic GABA receptor and it belongs to the large "Cys-loop ligand-gated ion channel" super-family.

Pollinator insects of the invention include species from the order *hymenoptera*, the family *Apidae* and the subfamily *Apinae* particularly the non-invasive species that does not damage crops or parasite other and/or native pollinator insects (insect native from a specific region), or damage hives.

Pollinator insects of the invention include in a non-limiting list: bees, honeybees such as *Apis mellifera, Apis cerana, Apis dorsata, Apis florea*, stingless bees such as *Melipona beecheii* or *Melipona yucatanica, Melipona quadrifasciata anthidioides,* orchid bees, bumble bees such as *Bombus franklini, Bombus terricola, Bombus affinis* and *Bombus occidentalis*. Preferably, the pollinator insect of the invention is a honey bee, and most preferably *Apis mellifera*.

In one embodiment of the invention, the isolated nucleic acid sequence comprises the Resistant to DieLdrin (RDL) subunit nucleic acid sequence as set forth in SEQ ID NO: 1 or variant thereof consisting of a nucleic acid sequence of less than 3400 bp and more than 1350 bp and having at least 90, 91, 92, 93, 94, 95, 96, 97, 98, 99, 99.1; 99.2; 99.3; 99.4; 99.5; 99.6; 99.7; 99.8; 99.9% of identity with SEQ ID NO: 1.

In one embodiment, a variation may occur in SEQ ID NO: 1.

In one embodiment of the invention, the isolated nucleic acid sequence comprises the GABA and glycine-like receptor of *Drosophila* (GRD) subunit nucleic acid sequence as set forth in SEQ ID NO : 3 or variant thereof consisting of a nucleic acid sequence of less than 2190 bp and more than 1395 bp and having at least 90, 91, 92, 93, 94, 95, 96, 97, 98, 99, 99.1; 99.2; 99.3; 99.4; 99.5; 99.6; 99.7; 99.8; 99.9% identity with SEQ ID NO: 3.

In one embodiment, a variation may occur in SEQ ID NO: 3.

In one embodiment of the invention, the isolated nucleic acid sequence comprises ligand-gated ion channel homologue 3 (LCCH3) subunit nucleic acid sequence as set forth in SEQ ID NO : 5 or variant thereof consisting of a nucleic acid sequence of more than 1470 bp and less than 1950 bp and having at least 90, 91, 92, 93, 94, 95, 96, 97, 98, 99, 99.1; 99.2; 99.3; 99.4; 99.5; 99.6; 99.7; 99.8; 99.9% identity with SEQ ID NO: 5.

In one embodiment, a variation may occur in SEQ ID NO: 5.

Another object of the present invention is a functional GABA-gated ion channel.

In one embodiment, the functional GABA-gated ion channel of the invention is a functional anionic channel.

In one embodiment, said functional anionic channel is selective for a chloride ($Cl^-$), a hydroxide ($OH^-$) or acetate, preferably a chloride anion.

In one embodiment, the functional anionic channel of the invention comprises the RDL subunit nucleic acid sequence as set forth in SEQ ID NO: 1 or variant thereof consisting of a nucleic acid sequence of less than 3400 bp and more than 1350 bp and having at least 90, 91, 92, 93, 94, 95, 96, 97, 98, 99, 99.1; 99.2; 99.3; 99.4; 99.5; 99.6; 99.7; 99.8; 99.9% of identity with SEQ ID NO: 1.

In another embodiment, the functional anionic channel of the invention comprises the RDL subunit nucleic acid sequence as set forth in SEQ ID NO: 1 or variant thereof consisting of a nucleic acid sequence of less than 3400 bp and more than 1350 bp and having at least 90, 91, 92, 93, 94, 95, 96, 97, 98, 99, 99.1; 99.2; 99.3; 99.4; 99.5; 99.6; 99.7; 99.8; 99.9% of identity with SEQ ID NO: 1 and the GRD subunit nucleic acid sequence as set forth in SEQ ID NO : 3 or variant thereof consisting of a nucleic acid sequence of less than 2190 bp and more than 1395 bp and having at least 90, 91, 92, 93, 94, 95, 96, 97, 98, 99, 99.1; 99.2; 99.3; 99.4; 99.5; 99.6; 99.7; 99.8; 99.9% identity with SEQ ID NO: 3.

In another embodiment, the functional anionic channel of the invention comprises the RDL subunit nucleic acid sequence as set forth in SEQ ID NO: 1 or variant thereof consisting of a nucleic acid sequence of less than 3400 bp and more than 1350 bp and having at least 90, 91, 92, 93, 94, 95, 96, 97, 98, 99, 99.1; 99.2; 99.3; 99.4; 99.5; 99.6; 99.7; 99.8; 99.9% of identity with SEQ ID NO: 1 and the LCCH3 subunit nucleic acid sequence as set forth in SEQ ID NO : 5 or variant thereof consisting of a nucleic acid sequence of more than 1970 bp and less than 1950 bp and having at least 90, 91, 92, 93, 94, 95, 96, 97, 98, 99, 99.1; 99.2; 99.3; 99.4; 99.5; 99.6; 99.7; 99.8; 99.9% identity with SEQ ID NO: 5.

In another embodiment, the functional anionic channel of the invention comprises the RDL subunit nucleic acid sequence as set forth in SEQ ID NO: 1 or variant thereof consisting of a nucleic acid sequence of less than 3400 bp and more than 1350 bp and having at least 90, 91, 92, 93, 94, 95, 96, 97, 98, 99, 99.1; 99.2; 99.3; 99.4; 99.5; 99.6; 99.7; 99.8; 99.9% of identity with SEQ ID NO: 1, the GRD subunit nucleic acid sequence as set forth in SEQ ID NO : 3 or variant thereof consisting of a nucleic acid sequence of less than 2190 bp and more than 1395 bp and having at least 90, 91, 92, 93, 94, 95, 96, 97, 98, 99, 99.1; 99.2; 99.3; 99.4; 99.5; 99.6; 99.7; 99.8; 99.9% identity with SEQ ID NO: 3 and the LCCH3 subunit nucleic acid sequence as set forth in SEQ ID NO : 5 or variant thereof consisting of a nucleic acid sequence of more than 1970 bp and less than 1950 bp and having at least 90, 91, 92, 93, 94, 95, 96, 97, 98, 99, 99.1; 99.2; 99.3; 99.4; 99.5; 99.6; 99.7; 99.8; 99.9% identity with SEQ ID NO: 5.

In another embodiment, the functional GABA-gated ion channel of the invention is a functional cationic channel.

In another embodiment, said functional cationic channel is selective for potassium ($K^+$), sodium ($Na^+$) or a tetraethylammonium ($TEA^+$) cation, preferably a sodium cation.

In another embodiment, the functional cationic channel of the invention comprises the GRD subunit nucleic acid sequence as set forth in SEQ ID NO : 3 or variant thereof consisting of a nucleic acid sequence of less than 2190 bp and more than 1395 bp and having at least 90, 91, 92, 93, 94, 95, 96, 97, 98, 99, 99.1; 99.2; 99.3; 99.4; 99.5; 99.6; 99.7; 99.8; 99.9% identity with SEQ ID NO: 3 and the LCCH3 subunit nucleic acid sequence as set forth in SEQ ID NO : 5 or variant thereof consisting of a nucleic acid sequence of more than 1970 bp and less than 1950 bp and having at least 90, 91, 92, 93, 94, 95, 96, 97, 98, 99, 99.1; 99.2; 99.3; 99.4; 99.5; 99.6; 99.7; 99.8; 99.9% identity with SEQ ID NO: 5.

Another object of the invention is an expression vector comprising at least one subunit of a GABA-gated ion channel of a pollinator insect and able to express said subunit of said GABA-gated ion channel in a suitable modified cell.

In one embodiment of the invention, the vector comprises the RDL subunit nucleic acid sequence as set forth in SEQ ID NO: 1 or variant thereof consisting of a nucleic acid sequence of less than 3400 bp and more than 1350 bp and having at least 90, 91, 92, 93, 94, 95, 96, 97, 98, 99, 99.1; 99.2; 99.3; 99.4; 99.5; 99.6; 99.7; 99.8; 99.9% of identity with SEQ ID NO: 1.

In another embodiment of the invention, the vector comprises the GRD subunit nucleic acid sequence as set forth in SEQ ID NO : 3 or variant thereof consisting of a nucleic acid sequence of less than 2190 bp and more than 1395 bp and having at least 90, 91, 92, 93, 94, 95, 96, 97, 98, 99, 99.1; 99.2; 99.3; 99.4; 99.5; 99.6; 99.7; 99.8; 99.9% identity with SEQ ID NO: 3.

In another embodiment of the invention, the vector comprises the LCCH3 subunit nucleic acid sequence as set forth in SEQ ID NO : 5 or variant thereof consisting of a nucleic acid sequence of more than 1970 bp and less than 1950 bp and having at least 90, 91, 92, 93, 94, 95, 96, 97, 98, 99, 99.1; 99.2; 99.3; 99.4; 99.5; 99.6; 99.7; 99.8; 99.9% identity with SEQ ID NO: 5.

In another embodiment of the invention, the vector comprises the RDL subunit nucleic acid sequence as set forth in SEQ ID NO: 1 or variant thereof consisting of a nucleic acid sequence of less than 3400 bp and more than 1350 bp and having at least 90, 91, 92, 93, 94, 95, 96, 97, 98, 99, 99.1; 99.2; 99.3; 99.4; 99.5; 99.6; 99.7; 99.8; 99.9% of identity with SEQ ID NO: 1 and the GRD subunit nucleic acid sequence as set forth in SEQ ID NO : 3 or variant thereof consisting of a nucleic acid sequence of less than 2190 bp and more than 1395 bp and having at least 90, 91, 92, 93, 94, 95, 96, 97, 98, 99, 99.1; 99.2; 99.3; 99.4; 99.5; 99.6; 99.7; 99.8; 99.9% identity with SEQ ID NO: 3.

In another embodiment of the invention, the vector comprises the RDL subunit nucleic acid sequence as set forth in SEQ ID NO: 1 or variant thereof consisting of a nucleic acid sequence of less than 3400 bp and more than 1350 bp and having at least 90, 91, 92, 93, 94, 95, 96, 97, 98, 99, 99.1; 99.2; 99.3; 99.4; 99.5; 99.6; 99.7; 99.8; 99.9% of identity with SEQ ID NO: 1 and the LCCH3 subunit nucleic acid sequence as set forth in SEQ ID NO : 5 or variant thereof consisting of a nucleic acid sequence of more than 1970 bp and less than 1950 bp and having at least 90, 91, 92, 93, 94, 95, 96, 97, 98, 99, 99.1; 99.2; 99.3; 99.4; 99.5; 99.6; 99.7; 99.8; 99.9% identity with SEQ ID NO: 5.

In another embodiment of the invention, the vector comprises the RDL subunit nucleic acid sequence as set forth in SEQ ID NO: 1 or variant thereof consisting of a nucleic acid sequence of less than 3400 bp and more than 1350 bp and having at least 90, 91, 92, 93, 94, 95, 96, 97, 98, 99, 99.1; 99.2; 99.3; 99.4; 99.5; 99.6; 99.7; 99.8; 99.9% of identity with SEQ ID NO: 1, the GRD subunit nucleic acid sequence as set forth in SEQ ID NO : 3 or variant thereof consisting of a nucleic acid sequence of less than 2190 bp and more than 1395 bp and having at least 90, 91, 92, 93, 94, 95, 96, 97, 98, 99, 99.1; 99.2; 99.3; 99.4; 99.5; 99.6; 99.7; 99.8; 99.9% identity with SEQ ID NO: 3 and the LCCH3 subunit nucleic acid sequence as set forth in SEQ ID NO : 5 or variant thereof consisting of a nucleic acid sequence of more than 1970 bp and less than 1950 bp and having at least 90, 91, 92, 93, 94, 95, 96, 97, 98, 99, 99.1; 99.2; 99.3; 99.4; 99.5; 99.6; 99.7; 99.8; 99.9% identity with SEQ ID NO: 5.

In another embodiment of the invention, the vector comprises the GRD subunit nucleic acid sequence as set forth in SEQ ID NO : 3 or variant thereof consisting of a nucleic acid sequence of less than 2190 bp and more than 1395 bp and having at least 90, 91, 92, 93, 94, 95, 96, 97, 98, 99, 99.1; 99.2; 99.3; 99.4; 99.5; 99.6; 99.7; 99.8; 99.9% identity with SEQ ID NO: 3 and the LCCH3 subunit nucleic acid sequence as set forth in SEQ ID NO : 5 or variant thereof consisting of a nucleic acid sequence of more than 1970 bp and less than 1950 bp and having at least 90, 91, 92, 93, 94, 95, 96, 97, 98, 99, 99.1; 99.2; 99.3; 99.4; 99.5; 99.6; 99.7; 99.8; 99.9% identity with SEQ ID NO: 5.

Expression vectors can be constructed by well-known molecular biological methods as described for example in Sambrook et al. (1989) Molecular Cloning: A Laboratory Manual Cold Spring Harbor Laboratory Press, Cold Spring Harbor, N.Y., or any of a myriad of laboratory manuals on recombinant DNA technology that are widely available. Expression vectors into which the nucleic acids of the present invention can be cloned under the control of a suitable promoter are also commercially available. Recombinant viral vectors, including retroviral, baculoviral, parvoviral and densoviral vectors are particularly preferred.

In one embodiment the expression vector comprises a strong constitutive or inducible promoter operably linked to the nucleic acid encoding the regulatory subunit of the GABA-gated ion channel. Suitable promoters are well known and readily available to those of ordinary skill in the art, and include for example, the heat shock promoter, metallothionein promoter, dexamethasone promoter, alcohol dehydrogenase promoter, the human cytomegalovirus immediate early promoter (CMV), the bacteriophage T7, T3 or SP6 promoters and the baculovirus promoters, i.e., the early promoter (e.g., IE-1 and etl), the late promoters (e.g., vp39 and p6.9), the very late promoters (e.g., polh and p10) and the hybrid promoter (e.g., vp39/polh).

Another object of the invention is a modified cell that expresses at least one vector described here above.

Modified cells as used herein refers to eukaryote cell. Modified cells are well known in the state of the art for genetic engineering. These cells include in a non-limiting list: prokaryotic cells, eukaryotic cells, in particular bacteria such as *Escherichia coli, Bacillus sp.*, or yeasts such as *Saccharomyces cerevisiae,* fungus such as *Aspergillus niger*, insect cells such as SF9, SL1, or mammalian cells such as CHO, HEK293, PER-C6, amphibians cells such as for example oocytes of Xenopus.

Technics for genetic engineering as used in the present application are well known by the person skilled in the art. These technics are described in Guide to Molecular Cloning Technics (Editors Berger SL and Kimmel AR 1987 Methods in Enzymology 152: 359-371).

Depending on the cell to be modified, the person skilled in the art can determine the technology needed for the introduction of the nucleotide sequences of the present application in the selected modified cell and the vector used.

Techniques for introducing the nucleic acid molecules into the modified cells may involve the use of expression vectors which comprise the nucleic acid molecules. These expression vectors (such as plasmids and viruses; viruses including bacteriophage) can then be used to introduce the nucleic acid molecules into suitable modified cells. For example, GABA-gated ion channel expression can be studied in Xenopus oocytes. DNA encoding the GABA-gated ion channel of a pollinator insect can be injected into the oocyte nucleus or transformed into the oocyte using a suitable vector, or mRNA encoding the said GABA-gated ion channel can be injected directly into the oocyte, in order to obtain expression of a functional GABA-gated ion channel in the oocyte.

Various methods are known in the art for introducing nucleic acid molecules into modified cells. One method is microinjection, in which DNA is injected directly into the nucleus of cells through fine glass needles (or RNA is injected directly into the cytoplasm of cells). Alternatively, DNA can be incubated with an inert carbohydrate polymer (dextran) to which a positively charged chemical group (DEAE, for diethylaminoethyl) has been coupled. The DNA sticks to the DEAE-dextran via its negatively charged phosphate groups. These large DNA-containing particles stick in turn to the surfaces of cells, which are thought to take them in by a process known as endocytosis. Some of the DNA evades destruction in the cytoplasm of the cell and escapes to the nucleus, where it can be transcribed into RNA like any other gene in the cell. In another method, cells efficiently take in DNA in the form of a precipitate with calcium phosphate. In electroporation, cells are placed in a solution containing DNA and subjected to a brief electrical pulse that causes holes to open transiently in their membranes. DNA enters through the holes directly into the cytoplasm, bypassing the endocytotic vesicles through which they pass in the DEAE-dextran and calcium phosphate procedures (passage through these vesicles may sometimes destroy or damage DNA). DNA can also be incorporated into artificial lipid vesicles, liposomes, which fuse with the cell membrane, delivering their contents directly into the cytoplasm. In an even more direct approach, used primarily with plant cells and tissues, DNA is absorbed to the surface of tungsten microprojectiles and fired into cells with a device resembling a shotgun.

Several methods of microinjection, electroporation, and liposome fusion, have been adapted to introduce nucleic acids into cells and are well known by the skilled artisan.

Further methods for introducing nucleic acid molecules into cells involve the use of viral vectors. Since viral growth depends on the ability to get the viral genome into cells, viruses have devised clever and efficient methods for doing it. One such virus widely used for protein production is an insect virus, baculovirus. Baculovirus attracted the attention of researchers because during infection, it produces one of its structural proteins (the coat protein) to spectacular levels. If a foreign gene were to be substituted for this viral gene, it too ought to be produced at high level. Baculovirus, like vaccinia, is very large, and therefore foreign genes must be placed in the viral genome by recombination. To express a foreign gene in baculovirus, the gene of interest is cloned in place of the viral coat protein gene in a plasmid carrying a small portion of the viral genome. The recombinant plasmid is cotransfected into insect cells with wild-type baculovirus DNA. At a low frequency, the plasmid and viral DNAs recombine through homologous sequences, resulting in the insertion of the foreign gene into the viral genome. Virus plaques develop, and the plaques containing recombinant virus look different because they lack the coat protein. The plaques with recombinant virus are picked and expanded. This virus stock is then used to infect a fresh culture of insect cells, resulting in high expression of the foreign protein. Various viral vectors have also been used to transform cells, such as bacteriophage, vaccinia virus, adenovirus, and retrovirus.

Another object of the invention is a modified cell expressing a vector comprising the acid nucleic sequences as described here above.

In one embodiment of the invention, the modified cell comprises an expression vector comprising the RDL subunit nucleic acid sequence as set forth in SEQ ID NO: 1 or variant thereof consisting of a nucleic acid sequence of less than 3400 bp and more than 1350 bp and having at least 90, 91, 92, 93, 94, 95, 96, 97, 98, 99, 99.1; 99.2; 99.3; 99.4; 99.5; 99.6; 99.7; 99.8; 99.9% of identity with SEQ ID NO: 1.

In another embodiment of the invention, the modified cell comprises an expression vector comprising the RDL subunit nucleic acid sequence as set forth in SEQ ID NO: 1 or variant thereof consisting of a nucleic acid sequence of less than 3400 bp and more than 1350 bp and having at least 90, 91, 92, 93, 94, 95, 96, 97, 98, 99, 99.1; 99.2; 99.3; 99.4; 99.5; 99.6; 99.7; 99.8; 99.9% of identity with SEQ ID NO: 1 and the GRD subunit nucleic acid sequence as set forth in SEQ ID NO : 3 or variant thereof consisting of a nucleic acid sequence of less than 2190 bp and more than 1395 bp and having at least 90, 91, 92, 93, 94, 95, 96, 97, 98, 99, 99.1; 99.2; 99.3; 99.4; 99.5; 99.6; 99.7; 99.8; 99.9% identity with SEQ ID NO: 3.

In another embodiment of the invention, the modified cell comprises an expression vector comprising the RDL subunit nucleic acid sequence as set forth in SEQ ID NO: 1 or variant thereof consisting of a nucleic acid sequence of less than 3400 bp and more than 1350 bp and having at least 90, 91, 92, 93, 94, 95, 96, 97, 98, 99, 99.1; 99.2; 99.3; 99.4; 99.5; 99.6; 99.7; 99.8; 99.9% of identity with SEQ ID NO: 1 and the LCCH3 subunit nucleic acid sequence as set forth in SEQ ID NO : 5 or variant thereof consisting of a nucleic acid sequence of more than 1970 bp and less than 1950 bp and having at least 90, 91, 92, 93, 94, 95, 96, 97, 98, 99, 99.1; 99.2; 99.3; 99.4; 99.5; 99.6; 99.7; 99.8; 99.9% identity with SEQ ID NO: 5.

In another embodiment of the invention, the modified cell comprises an expression vector comprising the RDL subunit nucleic acid sequence as set forth in SEQ ID NO: 1 or variant thereof consisting of a nucleic acid sequence of less than 3400 bp and more than 1350 bp and having at least 90, 91, 92, 93, 94, 95, 96, 97, 98, 99, 99.1; 99.2; 99.3; 99.4; 99.5; 99.6; 99.7; 99.8; 99.9% of identity with SEQ ID NO: 1, the GRD subunit nucleic acid sequence as set forth in SEQ ID NO : 3 or variant thereof consisting of a nucleic acid sequence of less than 2190 bp and more than 1395 bp and having at least 90, 91, 92, 93, 94, 95, 96, 97, 98, 99, 99.1; 99.2; 99.3; 99.4; 99.5; 99.6; 99.7; 99.8; 99.9% identity with SEQ ID NO: 3 and the LCCH3 subunit nucleic acid sequence as set forth in SEQ ID NO : 5 or variant thereof consisting of a nucleic acid sequence of more than 1970 bp and less than 1950 bp and having at least 90, 91, 92, 93, 94, 95, 96, 97, 98, 99, 99.1; 99.2; 99.3; 99.4; 99.5; 99.6; 99.7; 99.8; 99.9% identity with SEQ ID NO: 5.

In another embodiment of the invention, the modified cell comprises an expression vector comprising the GRD subunit nucleic acid sequence as set forth in SEQ ID NO : 3 or variant thereof consisting of a nucleic acid sequence of less than 2190 bp and more than 1395 bp and having at least 90, 91, 92, 93, 94, 95, 96, 97, 98, 99, 99.1; 99.2; 99.3; 99.4; 99.5; 99.6; 99.7; 99.8; 99.9% identity with SEQ ID NO: 3 and the LCCH3 subunit nucleic acid sequence as set forth in SEQ ID NO : 5 or variant thereof consisting of a nucleic acid sequence of more than 1970 bp and less than 1950 bp and having at least 90, 91, 92, 93, 94, 95, 96, 97, 98, 99, 99.1; 99.2; 99.3; 99.4; 99.5; 99.6; 99.7; 99.8; 99.9% identity with SEQ ID NO: 5.

Another object of the invention is the modified cells comprising the functional expression of a subunit of the GABA-gated ion channel of the pollinator insect.

In one embodiment, said modified cell expresses the isolated protein sequence of RDL subunit as set forth in SEQ ID NO: 2 or a variant thereof having at least 90, 91, 92, 93, 94, 95, 96, 97, 98, 99, 99.1; 99.2; 99.3; 99.4; 99.5; 99.6; 99.7; 99.8; 99.9% of identity with SEQ ID NO: 2.

In one embodiment, said modified cell expresses the isolated protein sequence of GRD subunit as set forth in SEQ ID NO : 4 or a variant thereof of more than 464 amino acids and having at least 90, 91, 92, 93, 94, 95, 96, 97, 98, 99, 99.1; 99.2; 99.3; 99.4; 99.5; 99.6; 99.7; 99.8; 99.9% of identity with SEQ ID NO: 4.

In one embodiment, said modified cell expresses the isolated protein sequence of LCCH3 subunit as set forth in SEQ ID NO : 6 or a variant thereof having at least 90, 91, 92, 93, 94, 95, 96, 97, 98, 99, 99.1; 99.2; 99.3; 99.4; 99.5; 99.6; 99.7; 99.8; 99.9% of identity with SEQ ID NO: 6.

In another embodiment, modified cell of the invention expresses the isolated protein sequence of the RDL subunit as set forth in SEQ ID NO: 2 or a variant thereof having at least 90, 91, 92, 93, 94, 95, 96, 97, 98, 99, 99.1; 99.2; 99.3; 99.4; 99.5; 99.6; 99.7; 99.8; 99.9% of identity with of SEQ ID NO: 2 and at least another subunit of said GABA-gated ion channel as described here above.

In another embodiment, modified cell of the invention expresses the isolated protein sequence of the RDL subunit as set forth in SEQ ID NO: 2 or a variant thereof having at least 90, 91, 92, 93, 94, 95, 96, 97, 98, 99, 99.1; 99.2; 99.3; 99.4; 99.5; 99.6; 99.7; 99.8; 99.9% of identity with of SEQ ID NO: 2 and the isolated protein sequence of GRD subunit as set forth in SEQ ID NO : 4 or a variant thereof of more than 464 amino acids and having at least 90, 91, 92, 93, 94, 95, 96, 97, 98, 99, 99.1; 99.2; 99.3; 99.4; 99.5; 99.6; 99.7; 99.8; 99.9% of identity with SEQ ID NO: 4.

In another embodiment, modified cell of the invention expresses the isolated protein sequence of the RDL subunit as set forth in SEQ ID NO: 2 or a variant thereof having at least 90, 91, 92, 93, 94, 95, 96, 97, 98, 99, 99.1; 99.2; 99.3; 99.4; 99.5; 99.6; 99.7; 99.8; 99.9% of identity with of SEQ ID NO: 2 and the isolated protein sequence of LCCH3 subunit as set forth in SEQ ID NO : 6 or a variant thereof having at least 90, 91, 92, 93, 94, 95, 96, 97, 98, 99, 99.1; 99.2; 99.3; 99.4; 99.5; 99.6; 99.7; 99.8; 99.9% of identity with SEQ ID NO: 6.

In another embodiment, modified cell of the invention expresses the isolated protein sequence of the RDL subunit as set forth in SEQ ID NO: 2 or a variant thereof having at least 90, 91, 92, 93, 94, 95, 96, 97, 98, 99, 99.1; 99.2; 99.3; 99.4; 99.5; 99.6; 99.7; 99.8; 99.9% of identity with of SEQ ID NO: 2, the isolated protein sequence of GRD subunit as set forth in SEQ ID NO : 4 or a variant thereof of more than 464 amino acids and having at least 90, 91, 92, 93, 94, 95, 96, 97, 98, 99, 99.1; 99.2; 99.3; 99.4; 99.5; 99.6; 99.7; 99.8; 99.9% of identity with SEQ ID NO: 4 and the isolated protein sequence of LCCH3 subunit as set forth in SEQ ID NO : 6 or a variant thereof having at least 90, 91, 92, 93, 94, 95, 96, 97, 98, 99, 99.1; 99.2; 99.3; 99.4; 99.5; 99.6; 99.7; 99.8; 99.9% of identity with SEQ ID NO: 6.

In another embodiment, modified cell of the invention expresses the isolated protein sequence of GRD subunit as set forth in SEQ ID NO : 4 or a variant thereof of more than 464 amino acids and having at least 90, 91, 92, 93, 94, 95, 96, 97, 98, 99, 99.1; 99.2; 99.3; 99.4; 99.5; 99.6; 99.7; 99.8; 99.9% of identity with SEQ ID NO: 4 and the isolated protein sequence of LCCH3 subunit as set forth in SEQ ID NO : 6 or a variant thereof having at least 90, 91, 92, 93, 94, 95, 96, 97, 98, 99, 99.1; 99.2; 99.3; 99.4; 99.5; 99.6; 99.7; 99.8; 99.9% of identity with SEQ ID NO: 6.

Another object of the invention is an *in vitro* method to determine the effect of a test compound on the activity of a GABA-gated ion channel of a pollinator insect comprising:

a. contacting modified cells described herein with at least one test compound,

b. measuring the effect of said test compound on the GABA-gated ion channel activity, and

comparing said effect to the effect without test compound, thereby determining a modulating activity of said GABA-gated ion channel.

Another object of the invention is an *in vitro* method to determine the effect of a test compound on the activity of a GABA-gated ion channel of a pollinator insect comprising:

a. contacting a modified cell described herein with at least one known GABA-gated ion channel modulator,
b. measuring the effect of said modulator on the GABA-gated ion channel activity,
c. contacting said modified cell described herein with at least one test compound and said at least one modulator,
d. measuring the effect of said modulator and test compound on the GABA-gated ion channel activity, and

comparing the effect measured in d) to the effect measured in b), thereby determining a modulating activity of said GABA-gated ion channel.

Examples of known modulators of GABA-gated ion channel include but are not limited to: GABA, 4-amino-cis-butenoic acid (CACA), muscimol.

In one embodiment of the invention, said known agonist of GABA-gated ion channel is applied for example from 1 ms to 10 seconds.

Another object of the invention is an *in vitro* method to determine the effect of a test compound on the activity of a GABA-gated ion channel of a pollinator insect comprising:

a. contacting a modified cell described herein with a standard reference,
b. measuring the effect of said standard reference on the GABA-gated ion channel activity,
c. contacting said modified cell described herein with at least one test compound and a standard reference,
d. measuring the effect of said standard reference and said at least one test compound on the GABA-gated ion channel activity, and

comparing the effect measured in d) to the effect measured in b), thereby determining a modulating activity of said GABA-gated ion channel.

In one embodiment, the standard reference is a compound known to modulate the GABA-gated ion channel activity of a pollinator insect of the invention.

Examples of a standard reference of GABA-gated ion channel include but are not limited to: GABA, 4-amino-cis-butenoic acid (CACA), muscimol.

In another embodiment of the invention, the standard reference is a compound known to modulate the GABA-gated ion channel activity of other species such as invasive species. Examples of invasive species include but are not limited to: introduced species, imported bees, species from the order *hymenoptera*, the family *Vespidae* and the subfamily *Vespinae* particularly Asian predatory wasp (*Vespa velutina*), spiders, parasites, endoparasites, ectoparasites from the class *Arachnida,* the family *Varroidae* such as: *Varroa jacobsoni, Varroa destructor, Varroa underwoodi, Varroa rindereri*.

In another embodiment, the standard reference comprises at least one compound known to modulate the GABA-gated ion channel.

In one embodiment, the standard reference is an agonist.

In another embodiment, the standard reference is an allosteric modulator.

In one embodiment, lethal dose of a test compound is added to the culture medium.

In another embodiment, sub-lethal dose of a test compound is added to the culture medium.

In another embodiment, minimal modulation dose of a test compound is added to the culture medium.

Examples of test compounds comprise phytosanitary product which include but are not limited to: insecticides, pesticides, drugs, veterinary drugs (such as veterinary drugs against parasites) that include in a non-limiting list derivatives or analogs of: fipronil, endosulfan, dieldrin, picrotoxin, phenylpyrazol compounds, polychlorocycloalkane compound, barbiturates, benzodiazepines, alcohols, lindance, cyclodiene.

Examples of test compounds also comprise but are not limited to compounds already known to be toxic on other species such as invasive species as described here above.

Examples of test compounds comprise molecules which include but are not limited to: siRNAs, shRNAs, antisense oligonucleotide, ribozymes or aptamers, ligands, agonist or antagonist of GABA-gated ion channels, antibodies or fragments thereof, diabodies modulating activity of a GABA-gated ion channel of a pollinator insect. These test compounds can also include derivatives, analogs of: neurotoxins such as tetrodotoxin, and batrachotoxin, toxins from plants, venom of insects, spiders, cones, mollusks, snails, vertebrates such as snakes, scorpion toxins, or any other natural products used in integrated pest management.

In one embodiment of the invention, said test compound is already known to modulate the GABA-gated ion channel activity of a pollinator insect.

In another embodiment of the invention, said test compound is not yet known to modulate the GABA-gated ion channel activity of a pollinator insect.

In another embodiment of the invention, said test compound is a new chemical entity.

In one embodiment of the invention, said test compound increases or decreases the functional expression of GABA-gated ion channel in the modified cell.

In one embodiment of the invention, said test compound induces or reduces the GABA-gated ion channel expression at the cell surface.

In one embodiment, said test compound modulates GABA-gated ion channel activity via RDL subunit.

In another embodiment, said test compound modulates GABA-gated ion channel activity via GRD subunit.

In another embodiment, said test compound modulates GABA-gated ion channel activity via LCCH3 subunit.

In another embodiment, said test compound modulates GABA-gated ion channel activity via RDL subunit and GRD subunit.

In another embodiment, said test compound modulates GABA-gated ion channel activity via RDL subunit and LCCH3 subunit.

In another embodiment, said test compound modulates GABA-gated ion channel activity via RDL subunit, via GRD subunit and LCCH3 subunit.

In another embodiment, said test compound modulates GABA-gated ion channel activity via GRD subunit and LCCH3 subunit.

Upon activation, the GABA-gated ion channel selectively conducts Cl- through its pore, resulting in hyperpolarization of the neuron. This causes an inhibitory effect on neurotransmission by diminishing the chance of a successful action potential occurring.

The ligand GABA is the endogenous compound that causes this receptor to open; once bound to GABA, the protein receptor changes conformation within the membrane, opening the pore in order to allow chloride anions ($Cl^-$) to pass down an electrochemical gradient. Because the reversal potential for chloride in most neurons is close to or more negative than the resting membrane potential, activation of GABA-gated ion tends to stabilize or hyperpolarize the resting potential, and can make it more difficult for excitatory neurotransmitters to depolarize the neuron and generate an action potential. The net effect is typically inhibitory, reducing the activity of the neuron. The GABA-gated ion channel opens quickly and thus contributes to the early part of the inhibitory post-synaptic potential (IPSP). Blocking the GABA-gated ion channel thus induce hyperexcitability which is deleterious for the pollinator insect. GABA-gated ion channels can conduct other anions with variable permeability ratios relative to $Cl^-$.

However, depending on the subunit, the GABA gated ion channel selectively conducts cations through its pore upon activation. Indeed, GRD and LCCH3 subunits allow the fluxes of cations through the pore which result in cell depolarization and induce action potentials. A cell has a resting potential of -70mV, once the membrane potential changes to -50mV, then the cell has been depolarized and is thus activated.

In one embodiment, said test compound modulates the activity of the GABA-gated ion channel of the invention.

In one embodiment, said test compound modifies the gating kinetics of the GABA-gated ion channel.

In another embodiment, said test compound modulates (increases or decreases) the hyperpolarization due to the activity of a pollinator insect GABA-gated ion channel.

In another embodiment, said test compound modulates (increases or decreases) the depolarization due to the activity of a pollinator insect GABA-gated ion channel.

In another embodiment, said test compound modulates (increases or decreases) the ion uptake by the GABA-gated ion channel.

In another embodiment, said test compound induces deleterious neuronal hyperexcitability.

In another embodiment, said test compound modulates (increases or decreases) the duration of closed states.

In another embodiment, said test compound modulates (increases or decreases) the duration of open states.

In another embodiment, said test compound modulates (increases or decreases) the activation and/or desensitization kinetics of GABA ion channel.

In another embodiment, said test compound affinity is modulated upon channel opening.

In another embodiment, said test compound affinity is modulated upon channel closed-state.

In another embodiment, said test compound is dependent of the functional states of the GABA-gated ion channel with preferred affinity for the close and/or open and/or desensitized states.

In another embodiment, said test compound modulates (increases or decreases) the sorting, the targeting or the translocation of the GABA-gated ion channel of the invention.

In another embodiment, said test compound modulates (increases or decreases) the stability of the GABA-gated ion channel of the invention.

In another embodiment, said test compound modulates the subunits assembly of the GABA-gated ion channel of the invention.

In one embodiment, the method of the invention is a cell-based assay.

In one embodiment, the method of the invention is a high-throughput assay.

In another embodiment, the method of the invention is an electrophysiological method.

In another embodiment, the method of the invention is a fluorometry or luminometry method.

The methods of the invention can be in conventional laboratory format or adapted for high throughput. The term "high throughput" (HTS) refers to an assay design that allows easy analysis of multiple samples simultaneously, and capacity for robotic manipulation. Another desired feature of high throughput assays is an assay design that is optimized to reduce reagent usage, or minimize the number of manipulations in order to achieve the analysis desired.

Methods for measuring the effect of a test compound on a GABA-gated ion channel are well known in the state of the art. For example, the person skilled in the art knows that electrophysiological measurements in modified cells expressing functional GABA-gated ion channel can be used to test a compound.

In one embodiment, measuring the effect of a test compound comprises measuring the activation kinetics of the GABA-gated ion channel.

In another embodiment, measuring the effect of a test compound comprises measuring the deactivation kinetics of the GABA-gated ion channel during removal of the agonist.

In another embodiment, measuring the effect of a test compound comprises measuring the desensitization kinetics of the GABA-gated ion channel.

In another embodiment, measuring the effect of a test compound comprises measuring the current amplitude for the selective ions of the GABA-gated ion channel.

In another embodiment of the invention, the *in vitro* method can be used to screen a compound that inhibits, prevents or stops the action of a compound known to intoxicate a pollinator insect through its GABA-gated ion channel subunits.

In one embodiment of the invention, the *in vitro* method determine the effect of a test compound to provide modulation reference patterns and databases of modulation reference patterns for a wide range of molecules. The reference patterns are then used for the identification and classification of test molecules. Evaluation of test compounds may be used to achieve different results.

Methods for the classification of compounds according to the spectral density signature of evoked changes in cellular electric potential are known to the person skilled in the art; see, e.g., US patent No 6,377,057. Thus, compounds are classified according to their effect on ion channels, changes in membrane potential and ionic currents, and the frequency content of action potentials that the compound(s) evoke in excitable cells.

The spectral density changes of such evoked membrane potential or action potential are a characteristic for each channel type that is modulated by the test compound. A pattern of spectral changes in membrane potential is determined by contacting a responsive cell with a test compound, and monitoring the membrane potential or ionic currents over time. These changes correlate with the effect of that compound, or class of compounds, on the ion channels of the responding cell. This pattern of spectral changes provides a unique signature for the compound, and provides a useful method for characterization of channel modulating agents. The effect of a compound on ion channels, and on the action potential of a living cell, can provide useful information about the classification and identity of the compound. Methods and means for extracting such information are of particular interest for the analysis of molecules, with specific applications in pharmaceutical screening, drug discovery, environmental monitoring, biowarfare detection and classification, and the like. Examples of whole cell-based biosensors are described in Gross et al., Biosensors and Bioelectronics 10 (1995), 553-567.

Another object of the invention is an *in vitro* method to determine the toxicity of a test compound on a pollinator insect comprising:

a. contacting modified cells described herein with at least one test compound,
b. measuring the effect of said at least one test compound on the GABA-gated ion channel activity, and

comparing the effect to the effect without test compound, thereby determining the toxicity on said GABA-gated ion channel.

In one embodiment of the invention, a test compound is considered as toxic once a modulating effect is measured on the GABA-gated ion channel of a pollinator insect.

In one embodiment, a test compound is considered as toxic once it modifies the gating kinetics of the GABA-gated ion channel.

In another embodiment, a test compound is considered as toxic once it modulates (increases or decreases) the amplitude of ion current of a pollinator insect GABA-gated ion channel.

In another embodiment, a test compound is considered as toxic once it modulates (increases or decreases) the desensitized state.

In another embodiment, a test compound is considered as toxic once it alters the activation kinetics or voltage dependence of either channel activation or desensitization.

In another embodiment, a test compound is considered as toxic once it induces deleterious neuronal hyperexcitability.

In another embodiment, a test compound is considered as toxic once its affinity is modulated (increased or decreased) upon channel opening.

In another embodiment, a test compound is considered as toxic once it modulated channel closed-state.

In another embodiment, a test compound is considered as toxic once it is dependent of the functional states of the GABA-gated ion channel with preferred affinity for the close and/or open and/or desensitized states.

In another embodiment, a test compound is considered as toxic once the sorting, the targeting or the translocation of the GABA-gated ion channel of the invention is modified.

In another embodiment, a test compound is considered as toxic once the stability of the GABA-gated ion channel of the invention is modified.

In another embodiment, a test compound is considered as toxic once the subunits assembly of GABA-gated ion channel of the invention is modified.

In one embodiment, the test compound is not toxic for the pollinator insects of the invention.

In another embodiment, the test compound is toxic for the pollinator insects of the invention.

In another embodiment, the test compound is toxic for invasive species as described here above while said test compound is not toxic for the pollinator insects of the invention.

In another embodiment, the test compound is toxic for insects spreading diseases to a human subject. Examples of such insects are mosquitoes. Examples of such diseases include but are not limited to: malaria, dengue fever, Japanese encephalitis, Ross River virus infection, Barmah Forest virus infection, Murray Valley encephalitis, yellow fever, West Nile Virus.

Another object of the invention is an *in vitro* method for screening compounds that modulate the GABA-gated ion channel activity of a pollinator insect that comprises:

a. contacting modified cells described herein with at least one test compound,
b. measuring the effect of said at least one test compound on the GABA-gated ion channel activity, and

comparing said effect to the effect without test compound, thereby determining a modulation of activity of said GABA-gated ion channel and classifying said compound as an agonist, antagonist, positive allosteric modulator, negative allosteric modulator, non-competitive channel blocker, open channel blocker.

Another object of the invention is an *in vitro* method for screening compounds that modulate the GABA-gated ion channel activity of a pollinator insect that comprises:

a. contacting modified cells described herein with at least one agonist,
b. measuring the effect of said agonist on the GABA-gated ion channel activity, and
c. contacting modified cells described herein with said agonist compound and at least one test compound,
d. measuring the effect of said agonist and said test compound on the GABA-gated ion channel activity, and

comparing the effect measured in d) to the effect measured in b), thereby determining a modulation of activity of said GABA-gated ion channel and classifying said compound as an agonist, antagonist, positive allosteric modulator, negative allosteric modulator, non-competitive channel blocker, open channel blocker.

Another object of the invention is an *in vitro* method to detect the binding of a test compound on the GABA-gated ion channel of a pollinator insect comprising:

a. contacting modified cells described herein with at least one test compound, and
b. measuring the binding of said test compound on the GABA-gated ion channel of a pollinator insect.

In one embodiment, the method of the invention determines the ability of a test compound to bind to the GABA-gated ion channel of a pollinator insect. The host cells that co-express nucleic acids encoding at least one of the GABA-gated ion channel subunits or preferably membranes or membrane fragments obtained from the host cells, are subjected to a radioligand binding assay utilizing [$3^{H}$] muscimol, [$^3$H] GABA, or other ligands which bind to the GABA recognition site. The host cells or membranes are incubated in a suitable buffer with an amount of [$^3$H] muscimol capable of being fully bound to the host cells or membranes, and with varied known amounts of a compound to be tested for its ability to bind to the GABA recognition site. The incubation is terminated, radioactivity that is not bound to the cells or membranes is separated from radioactivity that remains bound, and the amount of radioactivity that remains bound to the cells or membranes is determined.

If the test compound has the ability to bind to the GABA recognition site, a portion of the [$^3$H] muscimol that would bind to the membrane is competitively or non-competitively displaced and thus separable from the membrane. Thus, the amount of radioactivity that remains bound is proportional to the ability of the test compound to bind to the GABA recognition site.

For example, the method to detect the binding comprises the steps of:

a. contacting the host cell of the invention or membranes obtained from the host cells with [$^3$H]-muscimol in a known amount capable of binding to the known amount of host cells or membranes, and varied known amounts of a test compound for its ability to bind to the GABA recognition site for a time and under conditions suitable for a portion of said [$^3$H]-muscimol to bind to said host cells or membranes,
b. separating the cells or membranes from the [$^3$H]-muscimol that is not bound to the cells or membranes,
c. determining the GABA receptor binding of said compound.

Either host cells or membranes containing the GABA-gated ion channel of pollinator insect may be used in the present method. Membranes can be obtained from the host cells of the present invention by methods known in the art. In a representative method, the host cells are pelleted by centrifugation, and the pellets are frozen, for example at -80°C. The frozen pellet is then thawed, suspended in buffer, and gently homogenized, for example by about five strokes in a glass/Teflon homogenizer. Membranes are then pelleted one or more times by centrifugation, for example at 120,000 g for 30 minutes. The membrane pellet is resuspended in buffer, for example 10 mM phosphate buffer pH 7.4. The protein concentration of the membranes may be determined by methods known to those of ordinary skill in the art. The membrane suspensions may be frozen for later use.

For the radioligand binding assay of the invention, the host cells or membranes are combined in an aqueous solution with [$^3$H]-muscimol and the test compound. The aqueous solution may be a physiological buffer such as a saline solution including for example phosphate buffered saline, tris (hydroxymethyl) aminomethane (TRIS) or N-[2-Hydroxyethyl] piperazine-N-[2-ethanesulfonic acid] (HEPES). The [$^3$H]-muscimol is used in an amount capable of being fully bound to the amount of intact cells or membranes used in the assay. The ordinarily skilled artisan can determine the amount of [$^3$H]-muscimol that is bound to a particular amount of cells or membranes by determining standard dose-response curves as described for example by Limbird, Cell Surface Receptors: A Short Course on Theory and Methods, Martinus Nijhoff Publishing, Boston, 1986. For example, it has been determined herein that a final concentration of 5-10 nM [$^3$H]-muscimol is suitable for use with a membrane preparation having a protein content of about 125 μg.

The test compound is used in a known amount, and multiple assays are performed with varied known amounts in order to obtain a dose-response curve. A control assay is performed in which test compound is replaced by buffer. In one embodiment, the test compound is used at a concentration of from about 1 nM to about 100 μM. Those of ordinary skill in the art can determine suitable and convenient volumes of the components of the assay. For example, a convenient assay may be performed using 200 μL of membrane preparation containing 125 μg of protein, 100 μL of the 50 nM [$^3$H]-muscimol, 20 μL of test compound, and 680 μL of buffer.

The host cells or membranes, [$^3$H]-muscimol and test compound are incubated for a time and under conditions suitable to achieve binding of [$^3$H]-muscimol to the GABA-recognition site in the intact cells or membranes. In one embodiment, the incubation is conducted for 45 to 180 minutes at from 30°C to 4°C, respectively. Preferably, the incubation is conducted for about 90 minutes at about 4°C.

The incubation is terminated by separating the cells or membranes from the aqueous solution in which the incubation was conducted. The termination is accomplished by vacuum filtration on glass fiber filters followed by washing of filters with cold buffer.

The GABA receptor binding activity of the test compound is proportional to the amount of [$^3$H]-muscimol that is separated from the cells or membranes. The amount of [$^3$H]-muscimol that is separated is determined by measuring the radioactivity bound to the cells or membranes retained on the filter. The amount of radioactivity that is separated is compared to the amount of radioactivity that is separated when the same assay is conducted in the absence of test compound. Radioactivity may be measured by methods known in the art, for example by liquid scintillation spectrometry.

Radioactivity measurements are used to determine radioligand binding parameters such as $K_D$ and $IC_{50}$ which are, in turn, determined by kinetic and equilibrium analysis of saturation binding data. $K_D$ is the equilibrium dissociation constant which represents the concentration of ligand that half-maximally occupies the receptor at equilibrium. $IC_{50}$ is the molar concentration of the test compound needed to produce half-maximal displacement of [$^3$H]-muscimol from the GABA-gated ion channel of the invention. The $IC_{50}$ provides a direct measure of the ability of the test compound to bind to the GABA recognition site of the insect GABA receptor of the invention. Compounds having $IC_{50}$ values of about 100 μM or less in the method of the present invention are considered to interact with the GABA recognition site of the GABA-gated ion channel of a pollinator insect.

Another object of the invention is a kit comprising the vector of the invention or the modified cell expressing the GABA-gated ion channel of the invention and reagents for conducting any one of the above described methods of the invention.

Optionally the kit can comprise culture medium, recombinant nucleic acid sequences, reagents, standard reference compounds, etc. Such kit would typically comprise a compartmentalized carrier suitable to hold in close confinement at least one container. The carrier would further comprise reagents useful for performing said methods. The carrier may also contain a means for detection such as labeled enzyme substrates or the like. Instructions can be provided to detail the use of the components of the kit, such as written instructions, video presentations, or instructions in a format that can be opened on a computer (e.g. a diskette or CD-ROM disk). These instructions indicate, for example, how to use the cells to screen test compounds of interest (such as ionotropic drugs).

In addition, the present invention relates to an apparatus and array, respectively, for use in the methods and assays of the present invention described herein. For example, a cell-potential measurement apparatus having a plurality of microelectrodes and which may be used and/or adapted in accordance with the teaching of the present invention is described in European patent application EP 0 689 051.

Furthermore, international application WO98/54294 describes an apparatus and method for monitoring cells and a method for monitoring changes in cells upon addition of a compound to the cell's environment, comprising a device which includes an array of microelectrodes disposed in a cell culture chamber, upon which array a portion of cells adhere to the surfaces of the microelectrodes. The diameter of the cells is larger than the diameters of the microelectrodes. A voltage signal is applied across each of the microelectrodes and a reference electrode. Detection and monitoring of the signals resulting from the application of the voltage signal provides information regarding the electrical characteristics of the individual cells, including impedance (combined cell membrane capacitance and conductance), action potential parameters, cell membrane capacitance, cell membrane conductance, and cell/substrate seal resistance.

The present invention also relates to an automated Voltage-Clamp Screening System for Xenopus oocytes comprising HiClamp robot, USB video camera, HiClamp software, accessories, and consumables which is a well-known technics in the state of the art.

The HiClamp is a fully-automated all-in-one solution for high-throughput functional secondary screening of test compounds based on the standard Xenopus expression system. The HiClamp functionality is based on the use of a novel system in which the oocyte is exposed to a test solution by moving it physically into the solution of interest, whereas in a standard system the solution is applied on the cell.

## BRIEF DESCRIPTION OF THE DRAWINGS

**Figure 1** is a photograph of an agarose gel showing gene expression of the subunits of the GABA-gated ion channel of *Apis Mellifera* (Am) at different stages and in different tissues at day 7 (Abdomen, Brain, Leg and Antenna).

**Figure 2** represents an electrophysiological study following the expression of the different combination of GABA-gated ion channel subunits in oocytes.

**Figure 3** represents the ion selectivity of the different combinations of GABA-gated ion channel subunits expressed in oocytes.

**Figure 4** represents an electrophysiological study showing the pharmacology of the different combination of GABA-gated ion channel subunits expressed in oocytes.

**Figure 5** represents recordings of Single channel activity of GABA-gated ion channel.

**Figure 6** represents GABA-gated ionic currents on Amel-RDL subunit using high-throughput apparatus (Hi-Clamp).

## EXAMPLES

The present invention is further illustrated by the following examples.

### *Material and Methods*

Total RNAs purification from bees

Bees have been anesthetized at 4°C. Whole brains, legs, antennas and abdomens, have been rapidly dissected under a binocular and then stored on ice. Total RNAs have been purified from whole brains with the RNeasy Mini Kit (Qiagen). For the other tissues and for larva (Day7 and Day18), total RNAs have been purified with the RNAwiz reagent (Life Technologies). The tissues have been homogenized in 600 $\mu$l of RLT Buffer (Qiagen) or 4ml of RNAwiz (Life Technologies). The remaining of the procedure has been carried on following the instructions of the manufacturers. The final concentration of total RNAs have been determined by using a spectrophotometer (Biophotometer, Eppendorf), and RNA integrity has been checked by running an aliquot of RNA on an agarose gel. Total RNAs have then been stored at -80°C until use.

Reverse transcription

The first strand of the cDNAs has been obtained with the SuperScript II Reverse Transcriptase (Life Technologies) and with Oligo(dT)18 primers , and the cDNAs 5' end has been obtained with the First Choice RLM-RACE kit (Life Technologies) following the instructions of the manufacturer. 1 μl of the obtained cDNAs has been subjected to PCR amplification with the Herculase II fusion polymerase (Agilent Technologies). The first PCR of the RACE has been carried out following the instructions of the manufacturer. For the second PCR of the RACE and the others PCR, the temperature and the duration of the denaturation step (92-98°C, 20-60s), of the hybridization step (55-65°C, 20-60s) and of the extension step (68 or 72°C, 30s-3mn), together with the final concentration of DMSO (0 to 8%), have been empirically optimized for each couple of primers. The PCRs displayed on the **Figure 1** have been obtained with the following primers (**Table 1**), for Amel-RDL : amrdl-001S and amrdl-003AS (799pb), for Amel-GRD : amgrd-001S and amgrd-004AS (867pb), and for Amel-LCCH3: amlcch3-001S and amlcch3-003AS (1143pb). When appropriate, amplified fragments have been purified from agarose gel with the NucleoSpin Extract II kit (Macherey-Nagel), phosphorylated with the T4 kinase (Life Technologies) and ligated into the cloning vectors pBluescript-II SK (Agilent Technologies). Recombinant plasmids have been sequenced on both strands by Eurofins MWG Operon.

Amplification of the sequence of the Amel-GRD subunit

A 5' RACE has been carried out using the 5' Outer primer (supplied by the manufacturer) and amgrd-005AS for the first PCR and using the 5' Inner primer (supplied by the manufacturer) and amgrd-004AS for the second PCR. The obtained fragment has allowed the identification of the nucleotides 1 to 1181 (SEQ ID NO: 3). Another PCR realized with the primers amgrd-001S and amgrd-002AS has allowed the identification of the nucleotides 315 to 1874 (SEQ ID NO: 3).

Amplification of the sequence of the Amel-LCCH3 subunit

A 5' RACE has been carried out using the 5' Outer primer (supplied by the manufacturer) and amlcch3-004AS for the first PCR and using the 5' Inner primer (supplied by the manufacturer) and amlcch3-003AS for the second PCR. The obtained fragment has allowed the identification of the nucleotides 1 to 1469 (SEQ ID NO: 5). Another PCR realized with the primers amlcch3-001S and amlcch3-002AS has allowed the identification of the nucleotides 328 to 1798 (SEQ ID NO: 5).

Amplification of the sequence of the Amel-RDL subunit.

A 5' RACE has been carried out using the 5' Outer primer (supplied by the manufacturer) and amrdl-004AS for the first PCR and using the 5' Inner primer (supplied by the manufacturer) and amrdl-003AS for the second PCR. The obtained fragment has allowed the identification of the nucleotides 1 to 1067 (SEQ ID NO: 1). Another PCR realized with the primers amrd1-001S and amrdl-002AS has allowed the identification of the nucleotides 270 to 1597 (SEQ ID NO: 1).

Oligonucleotides/primers.

Oligonucleotides have been designed thanks to the public library (NCBI) that contains the sequences of the contigs of genomic DNA and the assembled cDNAs of Apis mellifera. Lyophilized oligonucleotides (Eurofins MWG Operon) have been resuspended at 100 μM in distilled water, aliquoted at 10 μM and stored at -20°C until use.

Table 1: List of primers used to identify GABA-gated ion channel subunits.

| SEQ ID NO: | Name | Sequence |
|---|---|---|
| 7 | PL014 | CTAGAGCGGCCGCGAGCTCAGCGATCTAGAGAAAGC TTGAGTTAACGAGCTAGCGAGGTACCGAC |
| 8 | PL015 | TTAAGTCGGTACCTCGCTAGCTCGTTAACTCAAGCTT TCTCTAGATCGCTGAGCTCGCGGCCGCT |

(continued)

| SEQ ID NO: | Name | Sequence |
|---|---|---|
| 9 | amgrd-005AS | CACGATCTGCAGTGGCTTCTCG |
| 10 | amgrd-004AS | CCAGAAGGAAACCCAAGACAGGAC |
| 11 | amgrd-001S | GAATTGATGAATGAATCAGGACGTATTG |
| 12 | amgrd-002AS | CGACCAGTCTCACGTGCCATTTG |
| 13 | amlcch3-004AS | GAACTGCCACTAACCAGACCTGATC |
| 14 | amlcch3-003AS | GGTAATGGAGACATCCTGAGATCTTG |
| 15 | amlcch3-001S | CATGCATCACAGGATGTGGTTGCAG |
| 16 | amlcch3-002AS | CTCAGAGGACATAAAATATCCAATAGATGGC |
| 17 | amrdl-004AS | CTTGACGTAGGAGATTTTCGGTAAC |
| 18 | amrdl-003AS | GTTCGTCGACGACATTAGGGTGGTC |
| 19 | amrdl-001S | CATGCTGAATGACGTCAACATCTCCG |
| 20 | amrdl-002AS | CTCTTCGCTTATTTCGCCTCCTCG |

Electrophysiology

*RNA preparation*

Plasmids coding for Amel-RDL, Amel-GRD or Amel-LCCH3 subunits of the GABA-gated ion channel, as obtained above, have been linearized at a restriction site localized in the 3' sequence just following the STOP codon using the appropriate restriction enzyme (Table 2). The reaction medium (volume of 50μl) contained 10 μg of plasmid, 3μl of the restriction enzyme (New England Biolabs France, Evry, France), 5μl of 10X reaction buffer provided by the manufacturer and $H_2O$ to 50μl. The reaction was then incubated for 3 hours at 37°C. The linearized plasmids were then purified using the NucleoSpin Extract II (Macherey-Nagel EURL, Hoerd, France) kit following manufacturer instructions, and resuspended at a concentration of 1μg/μl using deionized water (concentration was verified by measuring the OD at 260nm with a Biophotometer (Eppendorf France SAS, Le Pecq, France)). The efficiency of the linearization was also checked by agarose gel using 3μg of each plasmid.

RNA for each subunit was then obtained by *in vitro* transcription using the kit T3- or T7- mMessage mMachine (Life Technologies, Saint Aubin, France); following manufacturer recommendations (3-4 hours at 37°C). mRNA were then purified by using the RNeasy Mini Kit (Qiagen SAS, Courtaboeuf, France), and resuspended in desioned water at a concentration of 1μg/μl (verified by measuring the OD at 260nm with a Biophotometer (Eppendorf France SAS, Le Pecq, France). The length of each mRNA was also verified on agarose gel using 0.5μg of RNA. Each mRNA was then aliquoted at 2μl and stored at -20°C for further use.

Table 2: cDNA, vector, restriction enzyme and RNA polymerases used for in vitro transcription.

| cDNA SEQ ID | Vector | Enzyme | Polymerase |
|---|---|---|---|
| Amel-RDL | pBS-RDL4 | NotI | T7 |
| Amel-GRD | pBS- | NotI | T7 |
| Amel-LCCH3 | pBS- | NotI | T7 |

*Oocyte preparation*

The Xenopus laevis, female (from CRBM, UMR 5237, CNRS, Montpellier) was first anaesthetized by immersion using MS222 (ethyl-aminobenzoate methane sulfonate, ref A5040, Sigma) at 0.2% (pH=7) diluted in water during 20-30 minutes. Then a small 1-2 cm incision on the abdomen in between the midline and the lateral aspect of the abdomen was made through the fascia and muscle to visualize the oocytes. Fascia and muscle were picked up with forceps before cutting to avoid cutting the liver. Oocyte strands were then gently externalized cut and placed in a Petri dish filled with the OR2 solution. The incision was closed by suturing both the fascia and skin layer in two layers using surgical thread.

The Xenopus was then allowed to recover in dedicated tank water for 1-2 hours.

Oocytes were then washed 3 times with OR2 and transfered in a 50 ml Falcon tube filled 30 ml of OR2 supplemented with collagenase 1A (1mg/ml ; ref C9891 Sigma), and placed in an orbital shaker for 2-3 hours. The proper enzymatic digestion of the follicular cell layer was followed by inspection of the oocytes under a 30X binocular. After completion oocytes were washed 2-3 times with OR2 and 2 times with ND96S (Table 3). Nicely isolated stade VI oocytes were selected and collected in batches of 30 oocytes in 30mm Petri dishes for injection.

Table 3: OR2 and ND96S solution.

| **OR2** | | | **ND96S** | | |
|---|---|---|---|---|---|
| | mM | g/l | | mM | g/l |
| NaCl | 82.5 | 4.81 | NaCl | 96 | 5.6 |
| KCl | 2 | 0.15 | KCl | 2 | 0.15 |
| MgCl2 | 1 | 0.2 | $CaCl_2$ | 1.8 | 0.264 |
| Hepes | 5 | 1.19 | $MgCl_2$ | 1 | 0.2 |
| | | | HEPES | 5 | 1.19 |
| pH=7.2 (NaOH) | | | Pyruvate | 2.5 | 0.275 |
| | | | Gentamicym | 0.05 | 1ml |
| | | | pH=7.5-7.6 (NaOH) | | |

*RNA or cDNA injection*

RNA injection was performed using glass pipets (Clark Electromedical Instrument CG150T1) pulled using a sutter Inst P30 microelectrode puller, giving a final sharp tip a 2-5 μm. Under a binocular microscope, the pipette was then mounted on a micromanipulator and connected a homemade pressure-injection system. The pipette is first filled with the RNA mixture by backfilling the tip. The Tip is immersed in a drop (2 μL) of the RNA mixture, and filled by connecting the pipet, via the injection system, to vacuum. The 2 μl of RNA are taken by the pipet with special care to avoid any air bubble. Then a batch of 20-30 oocytes are injected individually, under visual inspection with the microscope, with 25-50 nl of RNA mixture using the injection apparatus. The pipette is changed for each RNA mixture. For RNA, the point of injection on the oocytes is the equatorial ring, for DNA injection, the point of injection is the middle of the black/brown animal pole.

The mixture of RNA that have been used here are

- Amel-RDL, Amel-GRD or Amel-LCCH3 alone,
- Amel-RDL+ Amel-GRD ; Amel-RDL + Amel-LCCH3, or Amel-GRD + Amel-LCCH3.

The final concentration of RNA was always 1μg/μl.

After injection, each batch of oocytes was placed again on the orbital shaker (1 rotation /2sec) for 2-5 days prior recording, with the incubation medium (ND96S) renewed daily.

*Electrophysiological measurements*

*Two electrodes voltage-clamp set-up*

A home-made recording chamber (50 μl) is placed under a stereomicroscope and connected to an array of 8 reservoirs (50 ml syringes) containing the various solutions. The flow of solution from each syringe can individually be automatically switched ON or OFF by micro-electrovalves connected to the voltage-clamp recording software version 9.0 of the pClamp program (Axon Inst., Molecular devices).

The chamber is electrically connected to the ground by Agar bridges. Clark capillaries (with filament, GC150F10) are bent at ~120°C under flame and subsequently immersed in 60mm Petri dishes filled with almost boiling agar (high gel strength) dissolved at 1% in 3M KCl (5-10 capillaries can be placed per dish). When immersed, these bridges are usually filled naturally (by capillarity) by the hot agar solution. After cooling, two Agarbridges are "dissected" from the agar and placed in the bath-electrode holder previously filled with 3M KCl. For two-electrodes voltage-clamp, voltage and current electrodes were pulled from Clark Electromedical Instrument borosilicate glass capillaries (GC150T-10) using a P-97 Sutter Instrument Company puller. They have a resistance of 0.5-2 MΩ when filled with 3M KCl.

In both two-electrodes voltage-clamp and single-channel recordings voltage protocols and current recordings are made using version 9.0 of the pClamp program (Axon Inst., Molecular devices) running on a PC computer connected, via Digidata 1200 interface to the Geneclamp 500 amplifier (Axon Instruments, Inc.) for two-electrodes voltage-clamp or to the Axopatch 200B amplifier (Axon Instruments, Inc.) for single channel recordings. Thus, voltage-command, sampling, acquisition and analysis are done using pClamp program. Additional analysis was performed using the Microsoft Excel software. All experiments are performed at room temperature (20-25 °C).

Typically, an oocyte, injected 2 to 5 days before with a given mixture of RNA is placed in the recording chamber filled with the desired solution (usually Na100). Junction potentials (typically less than 3-5 mV) at the two electrodes are first cancelled using the zero button of the amplifier in Na100 solution, and the electrodes resistance is checked before introduction into the oocyte (between 0.3 to 1.5 MΩ). Both electrodes are then impaled into the oocyte and the resting membrane potential is measured (typically -30/- 50 mV). The oocyte is then voltage-clamped usually at -80mV. The effect of GABA on expressed channels is then check by switching the perfusion system from on reservoir of control solution (i.e. ND96 or Na100) from one containing GABA at a given concentration (usually between 0.1 to 100 μM), or GABA + a test compound. Sometime, the effect of a test compound can be determined directly, by applying 50 μl of the compound directly to the bath (with the main perfusion stopped) at the final working concentration (usually 20-50 μM), after proper dilution from a stock solution (usually 500-10000 μM in water) into the desired recording solution. We assume that there is no dilution in the recording chamber which has a volume of around 50 μl.

Membrane currents are measured either at steady voltage as the difference between the current amplitudes before and during the application of GABA. These measures can also be done at different membrane potential (steady potential or voltage -ramps from - 80mV to +20 mV, with the change in membrane conductance followed by the modification in the slope of the current-voltage curve: current recorded during the voltage ramp, with the corresponding voltage axis; see Figure 3). In these conditions, washing-out of the test compound is done by switching-on the gravity-driven perfusion of the chamber with the same solution without test compound. Similar experiments are performed with oocytes injected with different GABA subunit RNA.

The tested compound can either decrease or increase GABA-induced current or modified other parameters, such as the channel kinetics, or channel selectivity for example. Such modifications can be considered as toxic for bees, thus revealing a potential toxicity of these products for bees.

Dose-response curves are obtained by measuring the GABA-induced current amplitude ($I_{GABA}$), as measured before, for different concentrations of GABA or the tested compounds. From these measured, the $I_{GABA}$ = f(log([GABA]) is constructed and a nonlinear regression using the following equation is performed.

$$I_{GABA} = I_{GABA}Max / (1+exp([GABA]-EC_{50})/h)$$

Where $I_{GABA}$ is the GABA-induced current, $I_{GABA}$MAX is the current amplitude recorded for a saturating GABA concentration, [GABA] are the various GABA concentrations perfused, "h" is a slope factor. For each GABA subunit combinations the EC50 and the "h" factor is then calculated (Figure 2-4).

Ionic selectivity is calculated by measuring the current reversal potentials of GABA-induced currents in recording solutions of different compositions varying in either the concentration of the extracellular anions or cations or the nature of the extracellular anions or cations (see Tables 4-9).

Current Reversal potential (Erev(X)) is then measured as the potential at which the GABA-induced current is 0 during voltage ramps. This measure is done after digital subtraction of traces recorded before and after GABA application. This potential is measured for each ionic condition (Na100 K100, TEA100, Li100, Cs100 or choline100 solutions) and eventually corrected for the junction potential.

Ionic permeability ratios of GABA-gated channels (permeability of cation/anion X relative to Na/Cl) permeability: $P_X/P_{Na}$) can be deduced for each cation X from the shift in reversal potentials Erev(X)-Erev(Na) using the Goldman-Hodgkin-Katz (GHK) equation:

$$Erev(X) - Erev(Na) = RT/zF\ Ln(P_X [X]_o / P_{Na} [Na]_o)$$

$$P_X/P_{Na} = [Na]_o/[X]_o\ e^{[(Erev(X)-Erev(Na))zF/RT]}$$

where $[X]_o$ is the extracellular concentration of ion X and R, T, z and F have their usual meanings (RT/F ~ 25.3 mV at 20 °C). (Figure 3).

All these measurements are usually performed on 3-15 different oocytes of each batch for each concentration or compounds, and the resulting values represents the average result between these oocytes. The statistical significance is tested using the student t-test at 5%.

Solutions used for reversal potential measurements.

For solutions with different Na concentrations, NaCl100 and TEACl100 were mixed according to the Na concentration. For solutions with different concentrations of Cl, NaCl100 and NaAcetate100 were mixed accordingly (Tables 4-9).

Table 4

| NaCl100 | |
|---|---|
| Products | Concentration (mM) |
| NaCl | 100 |
| KCl | 0 |
| $CaCl_2$ | 0 |
| $MgCl_2$ | 2 |
| HEPES | 5 |
| pH 7.2 (NaOH) | |

Table 5

| CholineCl100 | |
|---|---|
| Products | Concentration (mM) |
| CholineCl | 100 |
| KCl | 0 |
| $CaCl_2$ | 0 |
| $MgCl_2$ | 2 |
| HEPES | 5 |
| pH 7.2 (NaOH) | |

Table 6

| KCl100 | |
|---|---|
| Products | Concentration (mM) |
| NaCl | 0 |
| KCl | 100 |
| $CaCl_2$ | 0 |
| $MgCl_2$ | 2 |
| HEPES | 5 |
| pH 7.2 (KOH) | |

Table 7

| NaAcetateCl100 | |
|---|---|
| Products | Concentration (mM) |
| NaAcetate | 100 |
| KCl | 0 |

(continued)

| NaAcetateCl100 | |
|---|---|
| Products | Concentration (mM) |
| $CaCl_2$ | 0 |
| $MgCl_2$ | 2 |
| HEPES | 5 |
| pH 7.2 (NaOH) | |

Table 8

| NaOH100 | |
|---|---|
| Products | Concentration (mM) |
| NaOH | 100 |
| KCl | 0 |
| $CaCl_2$ | 0 |
| $MgCl_2$ | 2 |
| HEPES | 5 |
| pH 7.2 (MethSulfonate) | |

Table 9

| TEA Cl100 | |
|---|---|
| Products | Concentration (mM) |
| TEA Cl | 100 |
| KCl | 0 |
| $CaCl_2$ | 0 |
| $MgCl_2$ | 2 |
| HEPES | 5 |
| pH 7.2 (TEA OH) | |

***Results***

Example 1: Recordings of Single channel activity of GABA-gated ion channel

For single-channel recordings, after oocytes isolation, and RNA injection, at the time of recording the oocyte vitelline membrane was removed using forceps under a stereomicroscope after immersion in a hypertonic solution (200 mM NaCl, 10 mM HEPES). The oocyte was then placed in the recording chamber filled with a depolarizing solution (100 mM KCl, 5 mM HEPES, 10 mM EGTA, pH 7.2 adjusted with KOH; the osmolarity was ~250 mosmol) in order to drive the oocyte membrane potential close to 0 mV. Patch pipettes were pulled in several stage using a DMZ multistage puller , coated using Sylgard® under a stereomicroscope,and fire-polished to give pipette with a final resistance of 8-12 MΩ when filled with the pipette solution containing 100 mM NaCl, 5 mM HEPES and 100μM GABA with the pH adjusted to 7.2 with NaOH. Osmolarity was checked and was close to ~290 mosmol. The liquid junction potential was 1-3 mV and was thus neglected.

After gigaseal formation, cell-attached patch-clamp currents were recorded with an Axopatch 200B amplifier (Molecular Devices), low-pass filtered at 2 kHz and digitized at 10 kHz using a Digidata 1200 interface and stored on a computer using the Clampex software..The membrane potential was set at different levels (from -150 mV to +100 mV), and GABA-induced single channels opening were recorded in a "variable-length events" mode of the Clampex software. Off-line analysis of the currents was done with the Clampfit software (Molecular Devices). Well-resolved single channel openings were detected by a threshold analysis set at 50% of the elementary current. Channel conductance and open-time constants were calculated from Gaussian and multi-exponential fits of amplitude and open-time histograms, respectively, obtained at different voltages.

In Figure 5, the top trace show single channel openings of GABA channels recorded on oocytes injected with the Amel-RDL subunit at -50 mV and with 100μM GABA in the patch pipette. The bottom graph display the average amplitudes (square) of theses current recorded at different potential (from -110 mV to -30 mV). Channel conductance was then calculated as the slope of this current-voltage curve from the linear regression shown as a black line. In this case the Amel-RDL GABA channel conductance was 27 pS.

Example 2: GABA-gated ionic currents using high-throughput apparatus (Hi-Clamp)

Typical current recording from an oocyte injected with the Amel-RDL RNA. Oocytes were isolated and injected as described above. Three days after injection, they were placed individually on a 96 well plate. In the "solution plate" of the Hi-Clamp system different doses of GABA (0.1, 1, 3, 10, 30 and 100μM) and of GABA 100μM + Picrotoxin (at 0.01, 0.1, 1 and 10μM) in ND96 were added to the wells. The HI-Clamp then took oocytes one by one, voltage-clamped them at -40 mV, and placed then in each of these solutions, with intermediary 30 second washing periods in ND96. TOP traces show a current trace recoded during the successive passage of the oocyte in these different solution with intermediary washing (Figure 6).

The bottom-left graph represents the dose-response curve for GABA on a semi-log representation (with EC50 and slope factor) obtained on 7 such oocytes, while the bottom-right histogram depicted the effects of picrotoxin at different doses on the current induced by 100μM GABA (on 7 oocytes) (Figure 6).

## Claims

1. A nucleic acid sequence of the Resistant to DieLdrin (RDL) subunit as set forth in SEQ ID NO: 1 or variant thereof consisting of a nucleic acid sequence of less than 3400 bp and more than 1350 bp and having at least 90% identity with SEQ ID NO: 1.

2. A nucleic acid sequence of the $\gamma$-Aminobutyric acid (GABA) and glycine-like receptor of *Drosophila* (GRD) subunit as set forth in SEQ ID NO: 3 or variant thereof consisting of a nucleic acid sequence of less than 2190 bp and more than 1395 bp and having at least 90% identity with SEQ ID NO: 3.

3. A nucleic acid sequence of the ligand-gated ion channel homologue 3 (LCCH3) subunit as set forth in SEQ ID NO: 5 or variant thereof consisting of a nucleic acid sequence of more than 1470 bp and less than 1950 bp and having at least 90% identity with SEQ ID NO: 5.

4. A vector comprising at least one nucleic acid sequence as in anyone of claims **1** to **3**.

5. A modified cell expressing at least one vector of claim **4**.

6. A GABA-gated ion channel of a pollinator insect comprising a RDL subunit as set forth in SEQ ID NO: 1 or variant thereof consisting of a nucleic acid sequence of less than 3400 bp and more than 1350 bp and having at least 90% identity with SEQ ID NO: 1.

7. The GABA-gated ion channel according to claim **6** further comprising at least one other subunit of said channel, wherein said other subunit is GRD and/or LCCH3 having the sequences as in anyone of claims **2** to **3**.

8. A GABA-gated ion channel of a pollinator insect comprising the GRD and LCCH3 subunits having the sequences as in anyone of claims **2** to **3**.

9. A vector comprising the channel according to claim **6** or **7** or **8**.

10. A modified cell expressing a channel according to claim **6** or **7** or **8** or a vector according to claim **9**.

11. The modified cell according to claim **5** or **10**, wherein said cell is an oocyte of Xenopus.

12. An *in vitro* method to determine the effect of a test compound on the modulation of activity of a GABA-gated ion channel of a pollinator insect comprising:

   a. contacting modified cells according to anyone of claims **10** to **11** with at least one test compound,
   b. measuring the effect of said test compound on the channel activity, and

   comparing said effect to the effect without test compound or to the effect of a reference value, thereby determining a modulation of activity of said channel.

13. The method according to claim **12**, for determining the toxicity of a test compound on a pollinator insect.

14. An *in vitro* method for screening compounds that modulate the GABA-gated ion channel activity of pollinator insects comprising:

   a. contacting modified cells according to anyone of claims **10** to **11** with at least one test compound,
   b. measuring the effect of said compound on the GABA-gated ion channel activity, and

   comparing said effect to the effect without test compound or to the effect of a reference value, thereby determining a modulation of activity of said GABA-gated ion channel.

15. A kit comprising at least one vector according to claim **4** or **9** or a modified cell according to claims **5** or **10** to **11** and reagents.

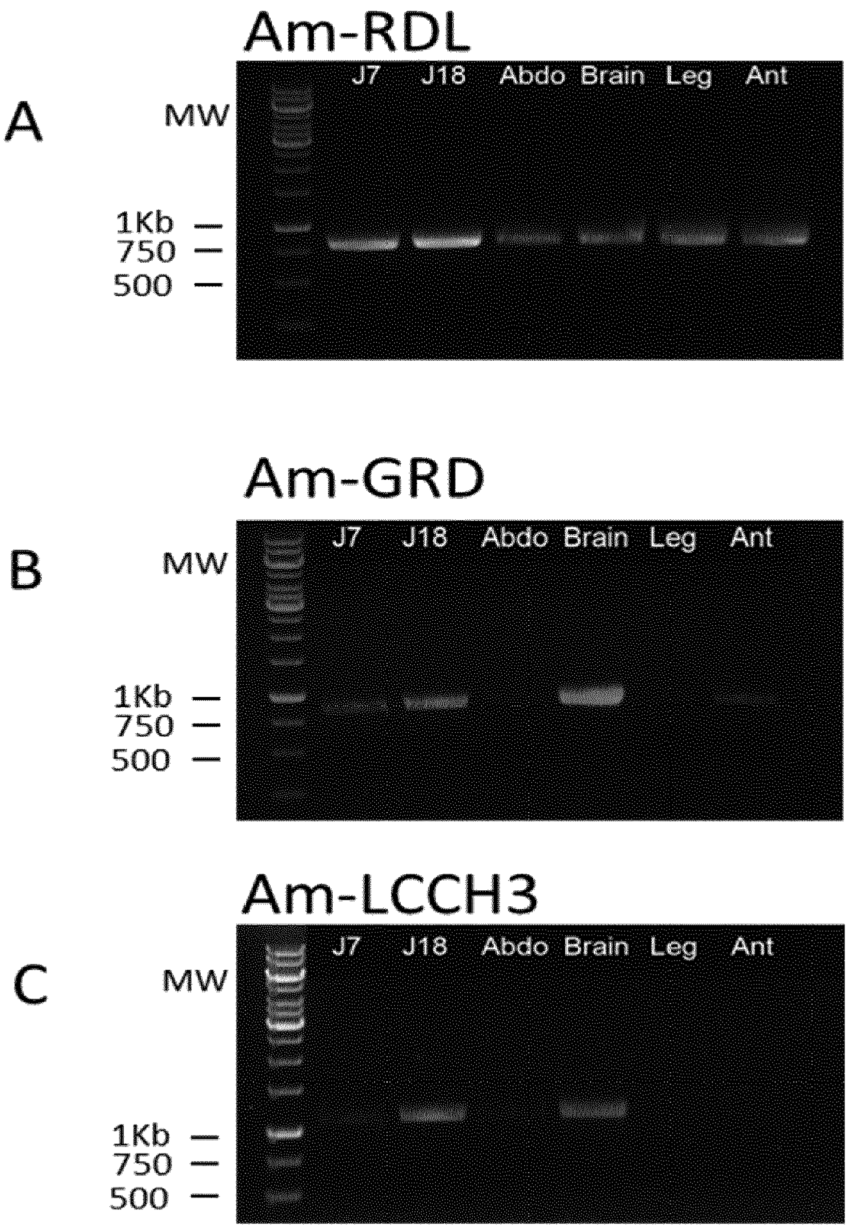
Am-RDL
A
MW
J7
J18
Abdo
Brain
Leg
Ant
1Kb
750
500
Am-GRD
B
MW
J7
J18
Abdo
Brain
Leg
Ant
1Kb
750
500
Am-LCCH3
C
MW
J7
J18
Abdo
Brain
Leg
Ant
1Kb
750
500

FIG. 1

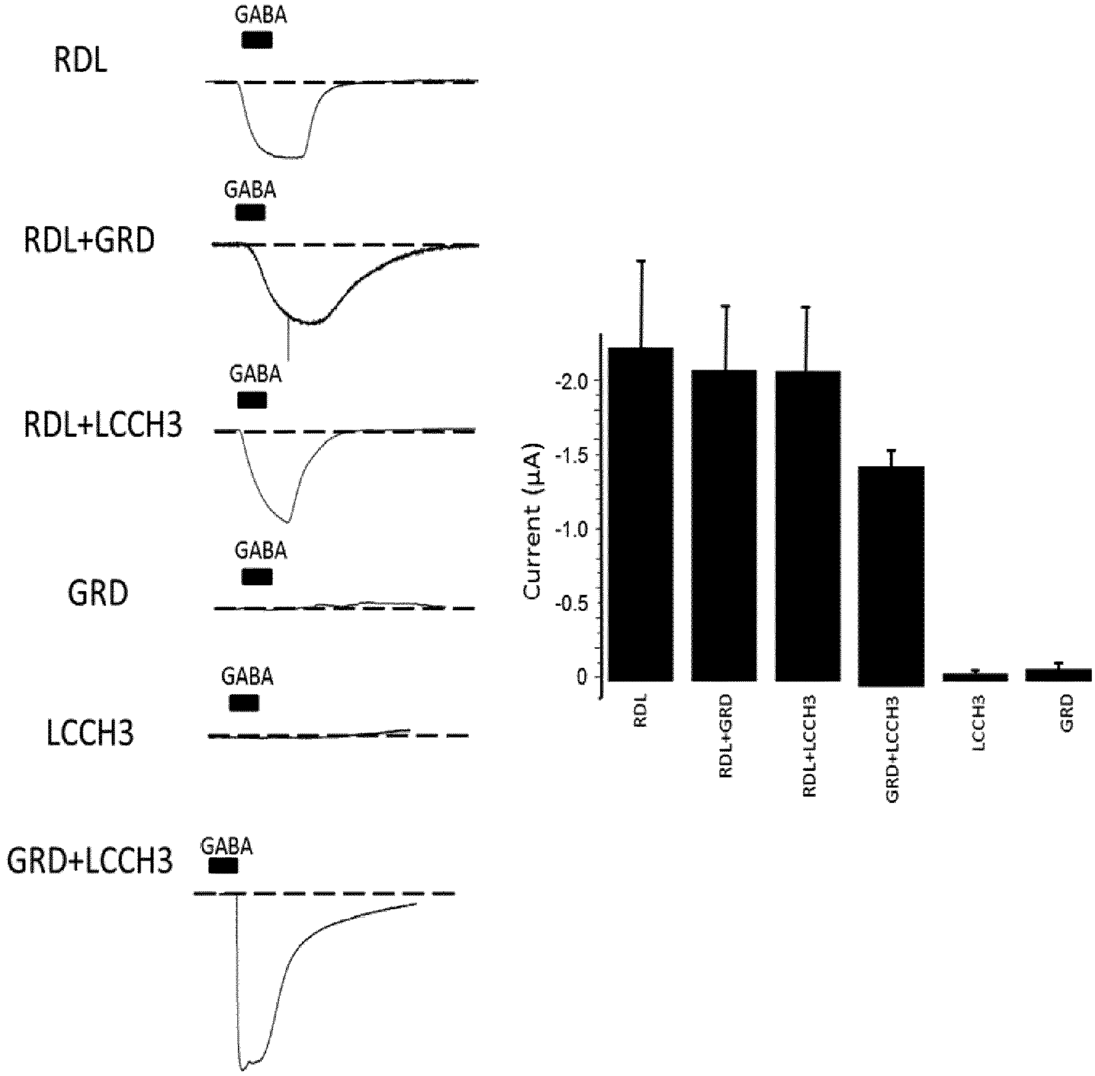


| | EC50 | Hill factor |
|---|---|---|
| RDL | 13μM | 1.8 |
| RDL+GRD | 15μM | 1.9 |
| RDL+LCCH3 | 15μM | 2 |
| GRD+LCCH3 | 32μM | 0.7 |

FIG. 2

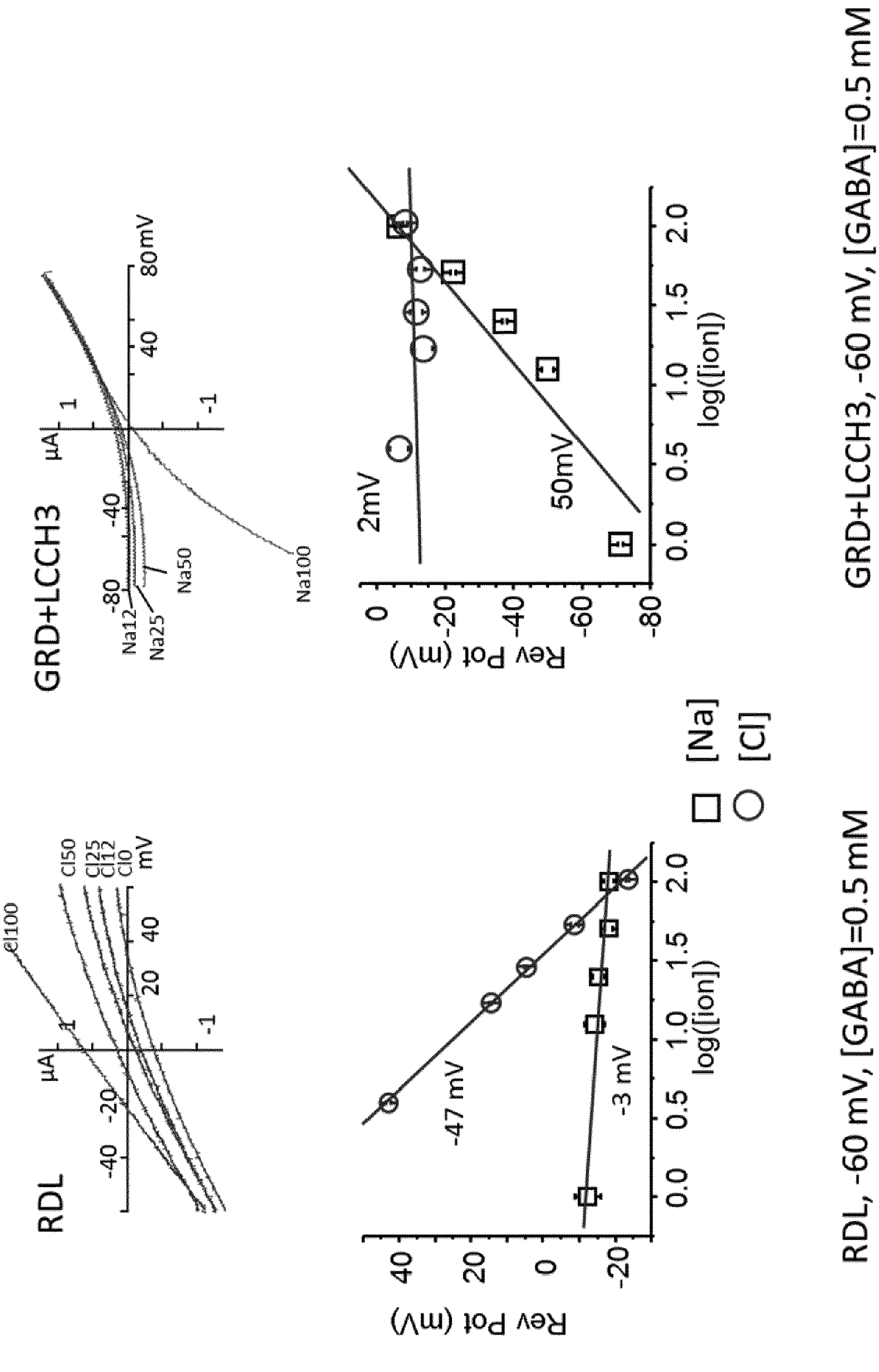
RDL
GRD+LCCH3
μA
mV
Cl100
Cl50
Cl25
Cl12
Cl0
Na12
Na25
Na50
Na100
80mV
Rev Pot (mV)
log([ion])
-47 mV
-3 mV
2mV
50mV
[Na]
[Cl]
RDL, -60 mV, [GABA]=0.5 mM
GRD+LCCH3, -60 mV, [GABA]=0.5 mM

FIG. 3

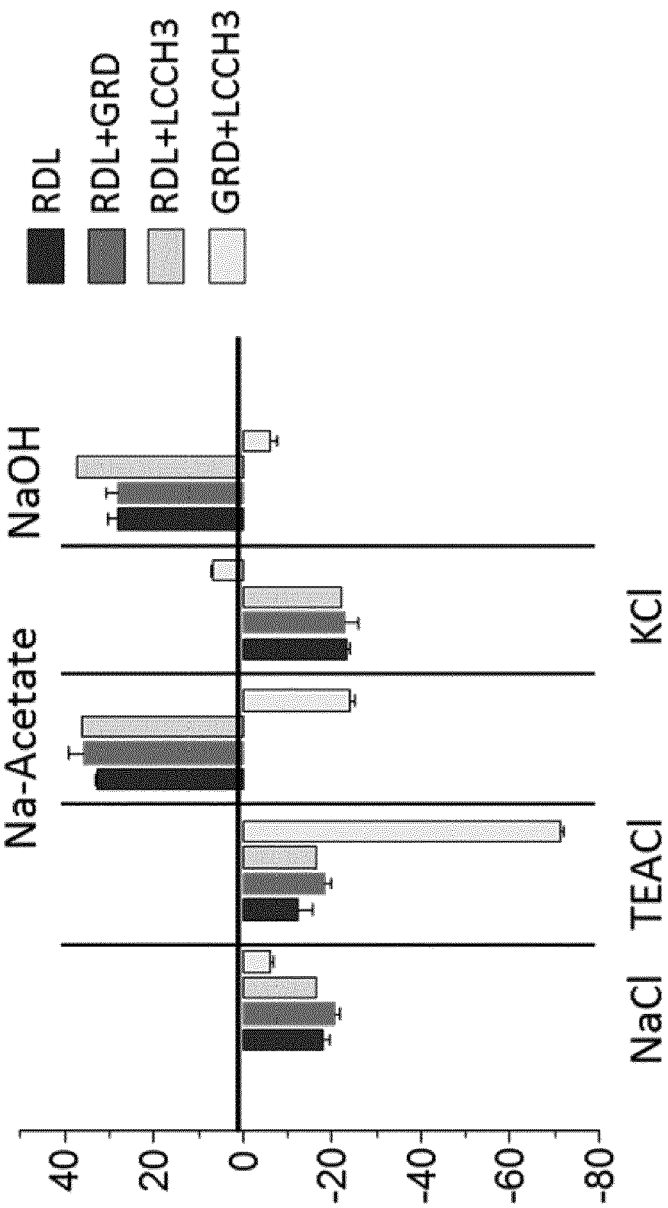
RDL
RDL+GRD
RDL+LCCH3
GRD+LCCH3
NaOH
Na-Acetate
KCl
TEACl
NaCl
Rev Pot (mV)
40
20
0
-20
-40
-60
-80

FIG. 3 (cont.)

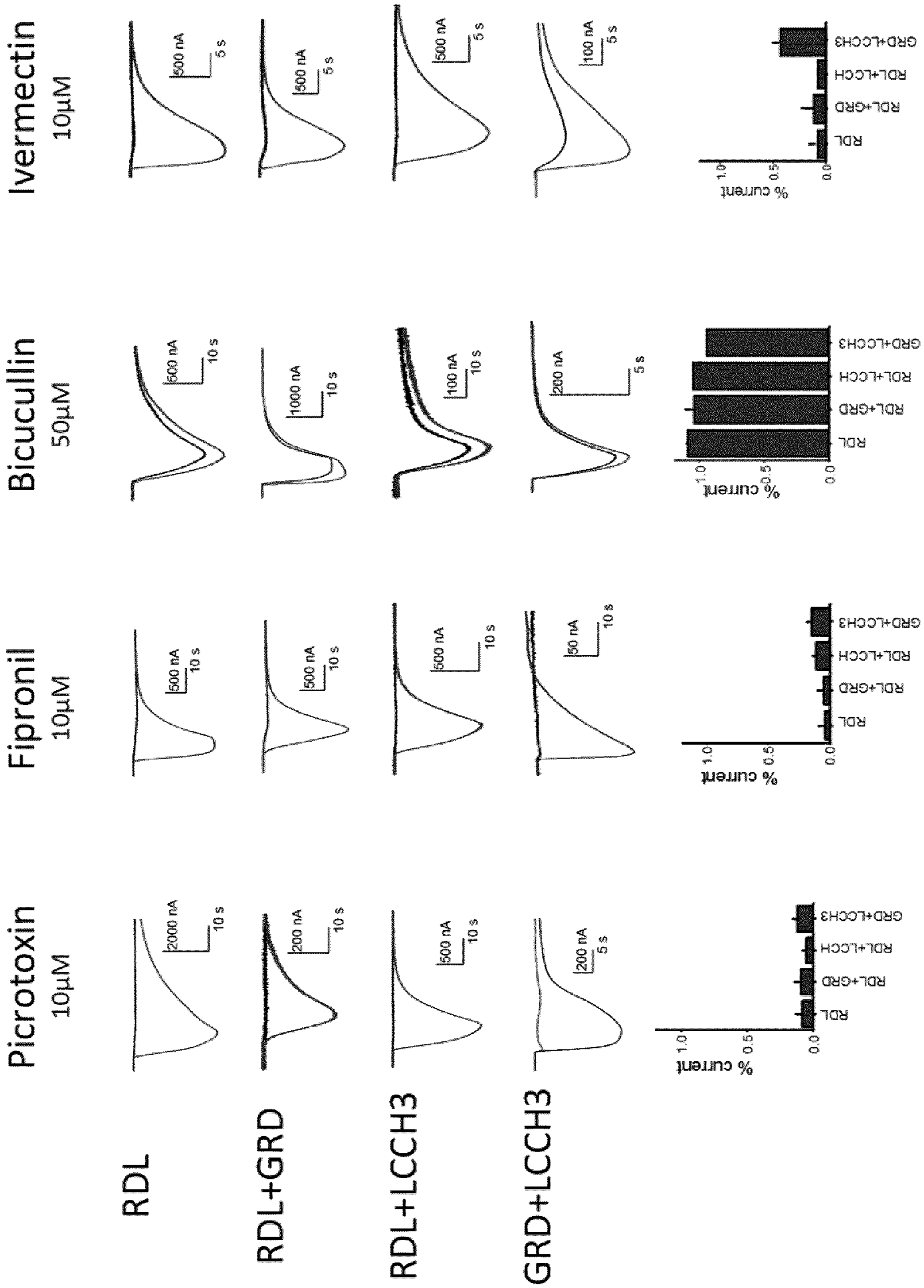
Picrotoxin
10µM
Fipronil
10µM
Bicucullin
50µM
Ivermectin
10µM
RDL
RDL+GRD
RDL+LCCH3
GRD+LCCH3
% current

FIG. 4

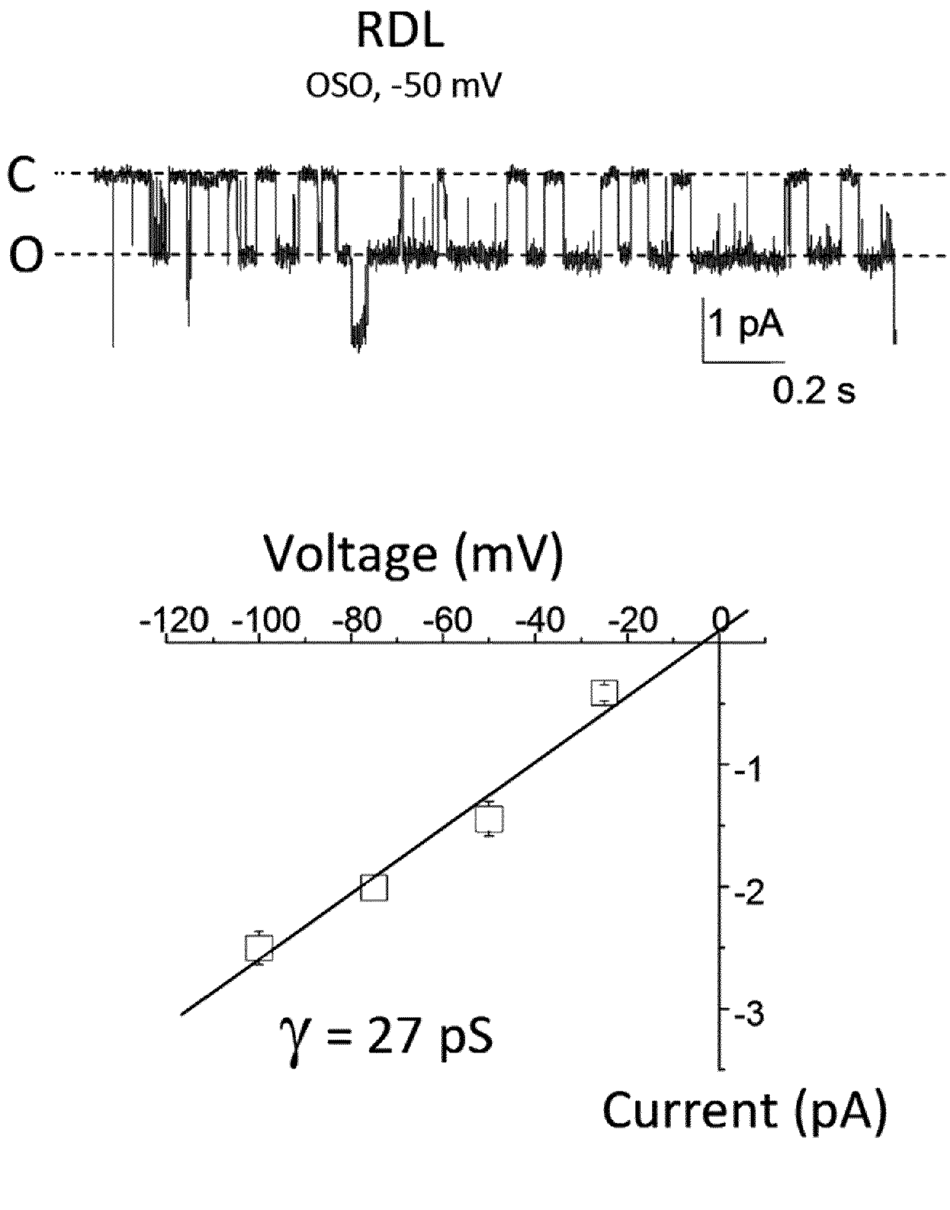
RDL
OSO, -50 mV
C
O
1 pA
0.2 s
Voltage (mV)
-120
-100
-80
-60
-40
-20
0
-1
-2
-3
γ = 27 pS
Current (pA)

FIG. 5

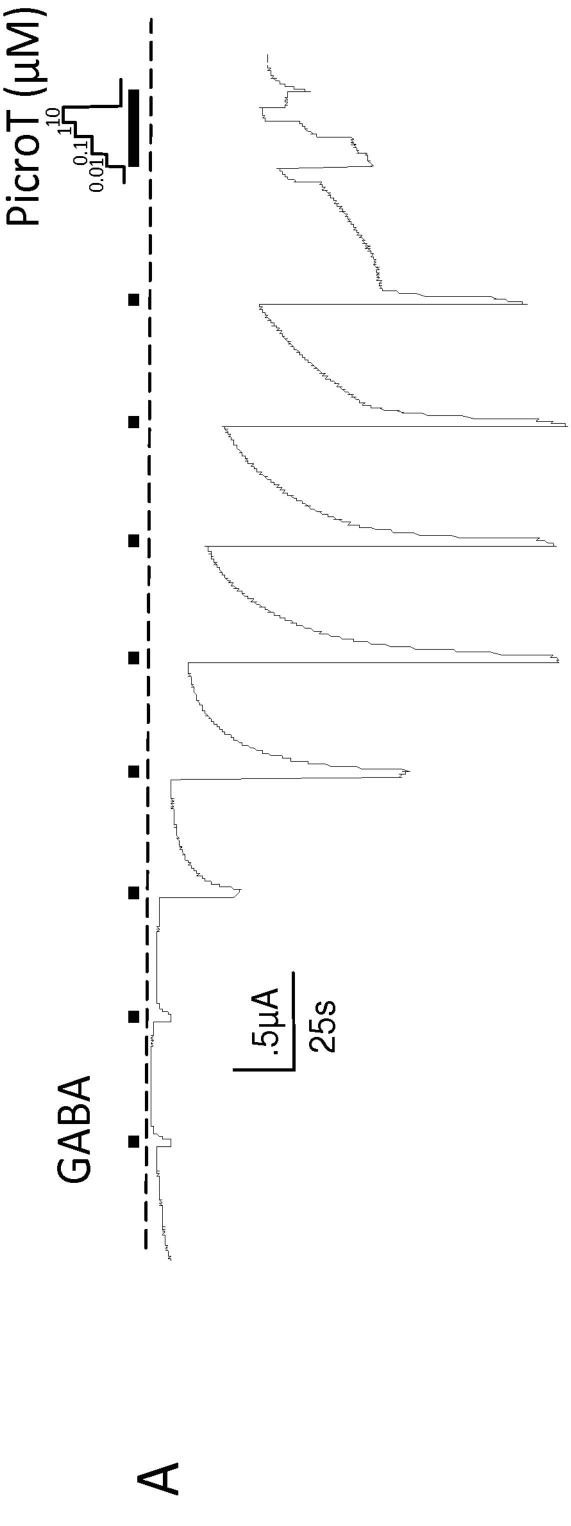


FIG. 6

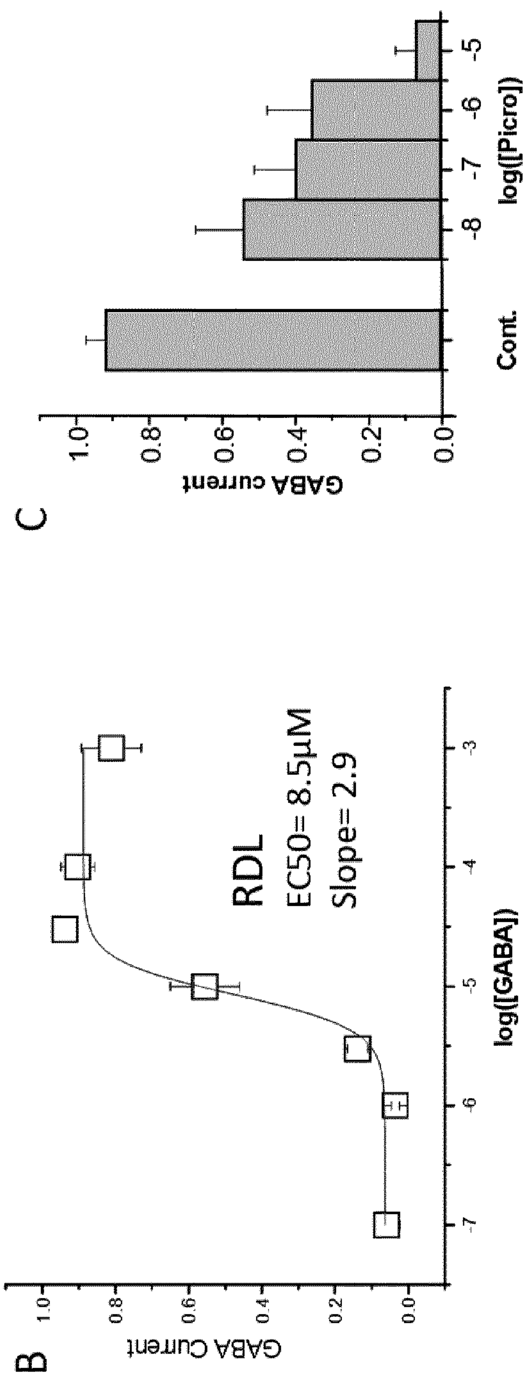
B
GABA Current
log([GABA])
RDL
EC50= 8.5μM
Slope= 2.9
C
GABA current
log([Picro])
Cont.

FIG. 6 (cont.)

Europäisches Patentamt
European Patent Office
Office européen des brevets

# EUROPEAN SEARCH REPORT

Application Number
EP 14 17 7633

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (IPC) |
| X | ANDREW K JONES ET AL: "The cys-loop ligand-gated ion channel superfamily of the honeybee, Apis mellifera", INVERTEBRATE NEUROSCIENCE, SPRINGER, BERLIN, DE, vol. 6, no. 3, 11 August 2006 (2006-08-11), pages 123-132, XP019387324, ISSN: 1439-1104, DOI: 10.1007/S10158-006-0026-Y<br>* abstract *<br>-& DATABASE EMBL [Online]<br><br>1 September 2006 (2006-09-01), "Apis mellifera GABA-gated chloride channel mRNA, partial cds, alternatively spliced.", XP002733653, retrieved from EBI accession no. EM_STD:DQ667181<br>Database accession no. DQ667181<br>* sequence *<br>-& DATABASE UniProt [Online]<br><br>3 October 2006 (2006-10-03), "SubName: Full=GABA-gated chloride channel {ECO:0000313¦EMBL:ABG75734.1}; Flags: Fragment;", XP002733654, retrieved from EBI accession no. UNIPROT:Q0GQR7<br>Database accession no. Q0GQR7<br>* sequence *<br>-/-- | 1-15 | INV.<br>C07K14/705<br><br>TECHNICAL FIELDS SEARCHED (IPC)<br>C07K |

The present search report has been drawn up for all claims

1

| Place of search | Date of completion of the search | Examiner |
|---|---|---|
| Munich | 12 January 2015 | Griesinger, Irina |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 14 17 7633

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| | -& DATABASE EMBL [Online]<br><br>1 September 2006 (2006-09-01), "Apis mellifera GABA-gated ion channel mRNA, partial cds.", XP002733655, retrieved from EBI accession no. EM_STD:DQ667183 Database accession no. DQ667183 * sequence *<br>-& DATABASE UniProt [Online]<br><br>3 October 2006 (2006-10-03), "SubName: Full=GABA-gated ion channel {ECO:0000313¦EMBL:ABG75735.1}; Flags: Fragment;", XP002733656, retrieved from EBI accession no. UNIPROT:Q0GQR6 Database accession no. Q0GQR6 * sequence *<br>-& DATABASE EMBL [Online]<br><br>1 September 2006 (2006-09-01), "Apis mellifera GABA-gated ion channel mRNA, complete cds.", XP002733657, retrieved from EBI accession no. EM_STD:DQ667184 Database accession no. DQ667184 * sequence *<br>-/-- | | TECHNICAL FIELDS SEARCHED (IPC) |

The present search report has been drawn up for all claims

1

| Place of search | Date of completion of the search | Examiner |
|---|---|---|
| Munich | 12 January 2015 | Griesinger, Irina |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 14 17 7633

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (IPC) |
| | -& DATABASE UniProt [Online]<br><br>3 October 2006 (2006-10-03),<br>"SubName: Full=GABA-gated ion channel {ECO:0000313¦EMBL:ABG75736.1};",<br>XP002733658,<br>retrieved from EBI accession no. UNIPROT:Q0GQR5<br>Database accession no. Q0GQR5<br>* sequence *<br>----- | | |
| X | WO 03/093789 A2 (FMC CORP [US]; CHEN RUIHUA [US]; KINNE LYLE P [US]; WRZESINSKI AMY [US) 13 November 2003 (2003-11-13)<br>* claims 6,13,19 *<br>----- | 1-15 | |
| X | DATABASE EMBL [Online]<br><br>18 October 2010 (2010-10-18),<br>"TSA: Apis mellifera isotig13604.Amelbrova mRNA sequence.",<br>XP002733650,<br>retrieved from EBI accession no. EM_TSA:HP497532<br>Database accession no. HP497532<br>* sequence *<br>----- | 1,4-6, 9-15 | TECHNICAL FIELDS SEARCHED (IPC) |
| X | DATABASE EMBL [Online]<br><br>30 April 2013 (2013-04-30),<br>"Nilaparvata lugens RDL protein (RDL) mRNA, complete cds.",<br>XP002733651,<br>retrieved from EBI accession no. EM_STD:KC841916<br>Database accession no. KC841916<br>* sequence *<br>-----<br>-/-- | 1,4-6, 9-15 | |

The present search report has been drawn up for all claims

1

| Place of search | Date of completion of the search | Examiner |
|---|---|---|
| Munich | 12 January 2015 | Griesinger, Irina |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 14 17 7633

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DATABASE EMBL [Online] 18 October 2010 (2010-10-18), "TSA: Apis mellifera isotig07569.Amelbrova mRNA sequence.", XP002733652, retrieved from EBI accession no. EM_TSA:HP491336 Database accession no. HP491336 * sequence * | 3-5,7-15 | |
| A | RAYMOND V ET AL: "NOVEL ANIMAL-HEALTH DRUG TARGETS FROM LIGAND-GATED CHLORIDE CHANNELS", NATURE REVIEWS. DRUG DISCOVERY, NATURE PUBLISHING GROUP, GB, vol. 1, 1 January 2002 (2002-01-01), pages 427-436, XP002491966, ISSN: 1474-1784, DOI: 10.1038/NRD821 * page 428 * | 1-15 | |

TECHNICAL FIELDS SEARCHED (IPC)

The present search report has been drawn up for all claims

1

| Place of search | Date of completion of the search | Examiner |
|---|---|---|
| Munich | 12 January 2015 | Griesinger, Irina |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 14 17 7633

This annex lists the patent family members relating to the patent documents cited in the above-mentioned European search report. The members are as contained in the European Patent Office EDP file on
The European Patent Office is in no way liable for these particulars which are merely given for the purpose of information.

12-01-2015

| Patent document cited in search report | Publication date | Patent family member(s) | Publication date |
|---|---|---|---|
| WO 03093789 A2 | 13-11-2003 | AU 2003225195 A1 | 17-11-2003 |
| | | EP 1581791 A2 | 05-10-2005 |
| | | JP 2005538699 A | 22-12-2005 |
| | | US 2006014219 A1 | 19-01-2006 |
| | | WO 03093789 A2 | 13-11-2003 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 6377057 B
- EP 0689051 A
- WO 9854294 A


### Non-patent literature cited in the description


- **SUCHAIL S et al.** *Environ Toxicol Chem,* 2001, vol. 20 (11), 2482-6
- **GELS JA.** *J Econ Entomol,* 2002, vol. 95 (4), 722-8
- **BARBARA et al.** *J Comp Physiol A Neuroethol Sens Neural Behav Physiol,* 2005, vol. 191, 823-836
- **GRÜNEWALD ; WERSING.** *J Comp Physiol A Neuroethol Sens Neural Behav Physiol,* 2008, vol. 194, 329-340
- **BLOOMQUIST.** *Arch Insect Biochem Physiol,* 2003, vol. 54, 145-156
- **RAYMOND ; SATTELLE.** *Nat Rev Drug Discov,* 2002, vol. 1, 427-436
- **SAMBROOK et al.** Molecular Cloning: A Laboratory Manual. Cold Spring Harbor Laboratory Press, 1989
- Guide to Molecular Cloning Technics. Methods in Enzymology. 1987, vol. 152, 359-371
- **GROSS et al.** *Biosensors and Bioelectronics,* 1995, vol. 10, 553-567
- **LIMBIRD.** Cell Surface Receptors: A Short Course on Theory and Methods. Martinus Nijhoff Publishing, 1986

SEQUENCE LISTING

<110> UniversitÃ© Montpellier II Science et Technique

<120> GABA CHANNEL SUBUNITS OF POLLINATOR INSECTS AND USES THEREOF

<130> CV - 333/EP

<160> 20

<170> BiSSAP 1.2

<210> 1
<211> 1597
<212> DNA
<213> Apis mellifera

<220>
<221> source
<222> 1..1597
<223> /mol_type="unassigned DNA"
/note="Resistant to DieLdrin subunit "
/organism="Apis mellifera"

<400> 1
aaaaacaacg cctcaggaca tccgctcgcg tcctcgccgc cgacgtcctt ctcgtcgtcc 60

tcgccgtggt cgtcgtcgtc gccgtggacg gcgccgcgac gacgttctct cttctcctcg 120

aacgtggaca ccgagcctcg tcccataagg atattaccat gtccttccac gccgcctcct 180

ggagcttcgc cctcctcgcg gccacggtcg cgttgctgcc cgccacgcat cgtgccccgt 240

tcgcgcaagc cgcaaccggc ggaggcagta tgctgaatga cgtcaacatc tccgcgatat 300

tggactcgtt ctccgtcagt tacgataaaa gagtaaggcc gaactacgga ggtcctcctg 360

tcgaggtggg cgtcaccatg tacgtcttga gtatcagctc gctatccgaa gtgaaaatgg 420

acttcacgct ggatttttac ttccggcaat tctggacgga tccacggctc gcgttcaaga 480

aacgtacggg ggtcgagacg ctgagcgtcg gctccgagtt catcaagaac atctgggtac 540

ccgacacgtt cttcgtcaac gagaagcagt cgtactttca tattgctacc accagcaacg 600

agttcattcg tattcatcac tcgggaagca tcacgcgtag tatacgttta acgatcacgg 660

catcgtgtcc gatgaatctg caatattttc caatggaccg acagctctgc catatcgaga 720

tcgagagctt cggatacacg atgagggaca tccggtacaa gtggaacgag ggaccgaaca 780

gcgtcggtgt cagcaacgag gtctccctgc ctcagttcaa ggtccttggt catcgccaac 840

gagccatgga gattagtcta actaccggaa attattcgcg gctggcctgc gagatccagt 900

tcgtacggtc gatgggctac tacctgattc aaatctacat accatcgggc ttgatcgtca 960

ttatatcgtg ggtgagcttt tggctgaacc gtaacgcgac cccccgctcgg gtcgccctcg 1020

gagtgaccac cgtgctcacc atgaccaccc taatgtcgtc gacgaacgcg gcgttaccga 1080

aaatctccta cgtcaagtcg atcgacgttt acctgggcac gtgtttcgtc atggtgttcg 1140

```
cgtcgttgct cgaatacgcg acggtgggct acatggcaaa gaggattcaa atgaggaaga    1200

accggttcca aaaaatcgcg gagagtatga agacggcgag ggagaaccca ggcccgccag    1260

gggtgcccgg tgatcacggc gatcacgcgc ctaagcaaac tgtgcggttc aaggtccacg    1320

acccgaaggc acacagcaag ggtggaacgc tcgagaacac tataaacggg cgggctgacg    1380

aggaggcagc gccggcgccg cagcatctta tccatcctgg gaaggatatc aacaagcttt    1440

acggtatgac gccatcggat atcgataaat actcccgcat cgtgtttccg gtatgcttcg    1500

tctgtttcaa cctaatgtac tggatcatct acctgcacat cagcgacgtc gtggcggacg    1560

atctcgtgct tctcgaggag gcgaaataag cgaagag                             1597
```

```
<210> 2
<211> 476
<212> PRT
<213> Apis mellifera

<220>
<223> Resistant to DieLdrin subunit

<400> 2
Met Ser Phe His Ala Ala Ser Trp Ser Phe Ala Leu Leu Ala Ala Thr
1               5                   10                  15
Val Ala Leu Leu Pro Ala Thr His Arg Ala Pro Phe Ala Gln Ala Ala
            20                  25                  30
Thr Gly Gly Gly Ser Met Leu Asn Asp Val Asn Ile Ser Ala Ile Leu
        35                  40                  45
Asp Ser Phe Ser Val Ser Tyr Asp Lys Arg Val Arg Pro Asn Tyr Gly
    50                  55                  60
Gly Pro Pro Val Glu Val Gly Val Thr Met Tyr Val Leu Ser Ile Ser
65                  70                  75                  80
Ser Leu Ser Glu Val Lys Met Asp Phe Thr Leu Asp Phe Tyr Phe Arg
                85                  90                  95
Gln Phe Trp Thr Asp Pro Arg Leu Ala Phe Lys Lys Arg Thr Gly Val
            100                 105                 110
Glu Thr Leu Ser Val Gly Ser Glu Phe Ile Lys Asn Ile Trp Val Pro
        115                 120                 125
Asp Thr Phe Phe Val Asn Glu Lys Gln Ser Tyr Phe His Ile Ala Thr
    130                 135                 140
Thr Ser Asn Glu Phe Ile Arg Ile His His Ser Gly Ser Ile Thr Arg
145                 150                 155                 160
Ser Ile Arg Leu Thr Ile Thr Ala Ser Cys Pro Met Asn Leu Gln Tyr
                165                 170                 175
Phe Pro Met Asp Arg Gln Leu Cys His Ile Glu Ile Glu Ser Phe Gly
            180                 185                 190
Tyr Thr Met Arg Asp Ile Arg Tyr Lys Trp Asn Glu Gly Pro Asn Ser
        195                 200                 205
Val Gly Val Ser Asn Glu Val Ser Leu Pro Gln Phe Lys Val Leu Gly
    210                 215                 220
His Arg Gln Arg Ala Met Glu Ile Ser Leu Thr Thr Gly Asn Tyr Ser
225                 230                 235                 240
Arg Leu Ala Cys Glu Ile Gln Phe Val Arg Ser Met Gly Tyr Tyr Leu
                245                 250                 255
Ile Gln Ile Tyr Ile Pro Ser Gly Leu Ile Val Ile Ile Ser Trp Val
            260                 265                 270
Ser Phe Trp Leu Asn Arg Asn Ala Thr Pro Ala Arg Val Ala Leu Gly
        275                 280                 285
Val Thr Thr Val Leu Thr Met Thr Thr Leu Met Ser Ser Thr Asn Ala
```

```
    290                 295                 300
Ala Leu Pro Lys Ile Ser Tyr Val Lys Ser Ile Asp Val Tyr Leu Gly
305                 310                 315                 320
Thr Cys Phe Val Met Val Phe Ala Ser Leu Leu Glu Tyr Ala Thr Val
                325                 330                 335
Gly Tyr Met Ala Lys Arg Ile Gln Met Arg Lys Asn Arg Phe Gln Lys
            340                 345                 350
Ile Ala Glu Ser Met Lys Thr Ala Arg Glu Asn Pro Gly Pro Pro Gly
        355                 360                 365
Val Pro Gly Asp His Gly Asp His Ala Pro Lys Gln Thr Val Arg Phe
    370                 375                 380
Lys Val His Asp Pro Lys Ala His Ser Lys Gly Gly Thr Leu Glu Asn
385                 390                 395                 400
Thr Ile Asn Gly Arg Ala Asp Glu Glu Ala Ala Pro Ala Pro Gln His
                405                 410                 415
Leu Ile His Pro Gly Lys Asp Ile Asn Lys Leu Tyr Gly Met Thr Pro
            420                 425                 430
Ser Asp Ile Asp Lys Tyr Ser Arg Ile Val Phe Pro Val Cys Phe Val
        435                 440                 445
Cys Phe Asn Leu Met Tyr Trp Ile Ile Tyr Leu His Ile Ser Asp Val
    450                 455                 460
Val Ala Asp Asp Leu Val Leu Leu Glu Glu Ala Lys
465                 470                 475
```

<210> 3
<211> 1874
<212> DNA
<213> Apis mellifera

<220>
<221> source
<222> 1..1874
<223> /mol_type="unassigned DNA"
/note="glycine-like receptor of Drosophila subunit "
/organism="Apis mellifera"

<400> 3

```
gaaaacgagg cagccagtac gcgtcgtggc ttcgccacgt gcttctcgtc ccgttggaaa      60

tttcgcgttc attatttgtc tgattatgtt ttcattttct tcgtcgtatt tttgttgcgt     120

taacttatca acttatgatg cacttccgct atgtgattaa ttaaaatctt aatattttcg     180

caatcgttga taactacgct cttgtgacca tgtgaccaat acatgtcatg tatttgctat     240

ggatattttt gcgtatacat tttgagcaaa cataatagaa atagaagaaa tctaaaacgc     300

tggccgtttc aaacgaattg atgaatgaat caggacgtat tgtgagggag gatcagtccc     360

aagacatgat gaagcagcga gtccttctgg ccctcgccaa cttgatcaca tcttatgcat     420

cgtttctagc ggcgacaggg gcgaaaacat ccacgaggca gaggacacag agcaataatc     480

atagcaacat aagcgagctt ttggacaatt tgcttcgcgg ttacgacaac agcgtgagac     540

cagacttcgg tggtccaccg gccacggttg aggtcgacat catggtgcgc agtatgggtc     600

cgatttccga agtcgatatg acttattcaa tggattgtta cttccggcaa tcgtgggtgg     660

atcgacgttt ggcttttcaa ggcggtaaag aaactctggc gcttagtata tcgatgctgg     720

cgaggatctg gaagccggat acatactttt acaacgggaa gcacagttat ttacacacta     780
```

```
taaccagtcc caataaattc gtcagacttt atcaagatgg tagagtgctc tacagttcaa     840
gactcacgat caaggcagga tgtccaatga atcttgaaaa ttttccaatg gatacacaaa     900
gatgtccact acaatttggt agcttcggtt acacgaagcg ggacgtgatc tacaagtgga     960
acagcgctcg acaagtagca atcgccgagg acatgaaatt atcccaattc gatttagttg    1020
ccaatccgac tgcaaattat tctgcatcga cgaccctttc acacgcggaa tattcgatgt    1080
tgctggtata ttttcatctg cagaggcata tgggtaattt tcttatacaa gtatatggac    1140
cttgtgtttt gctagtcgtc ctgtcttggg tttccttctg gttaaatcga gaagccactg    1200
cagatcgtgt atcattagga ataacaaccg tgttaacgat gacattttg ggattggagg     1260
cgcgaaccga tctgccaaaa gttccttacc ccactgcctt ggatttcttc gttttcctct    1320
cgttcgcctt cattttcgcc accattatac aattcgcagt ggtccattat ttcaccaagt    1380
acggttctgg cgagtgttat ttcagttcgg atctgagcga aagtgaaact tccagcgatg    1440
aagaggaagc ggatacgcgg ccattgaaga aagaaccaat ttcccaatcg aacaaaagaa    1500
ctgccggaaa cttagcaact cgtgaacatc caagtcgtaa tttcacaatg agtgaagacg    1560
gtatgatcga ggtaataccg ttatcagcaa ttccagaacc gagcaaaaat cctgcaatgg    1620
gtcactggca aatgtcctgc gtggcctgta gtccacctcc tcgtcaaact cctccaagca    1680
gaaaggaatc gggtcgaaga cgacgcagga ggactcctag atacaattct gtatcaaaaa    1740
tcgatcgagc cagccgcatc gtgttccctc tattcttcct agcgatcaac gtattttatt    1800
ggtttgcata cttgtcaaga agcgagcgta tcaattatta caacgtgaat tcaaatggca    1860
cgtgagactg gtcg                                                      1874
```

<210> 4
<211> 514
<212> PRT
<213> Apis mellifera

<220>
<223> glycine-like receptor of Drosophila

<400> 4

```
Met Asn Glu Ser Gly Arg Ile Val Arg Glu Asp Gln Ser Gln Asp Met
1               5                   10                  15
Met Lys Gln Arg Val Leu Leu Ala Leu Ala Asn Leu Ile Thr Ser Tyr
            20                  25                  30
Ala Ser Phe Leu Ala Ala Thr Gly Ala Lys Thr Ser Thr Arg Gln Arg
        35                  40                  45
Thr Gln Ser Asn Asn His Ser Asn Ile Ser Glu Leu Leu Asp Asn Leu
    50                  55                  60
Leu Arg Gly Tyr Asp Asn Ser Val Arg Pro Asp Phe Gly Gly Pro Pro
65                  70                  75                  80
Ala Thr Val Glu Val Asp Ile Met Val Arg Ser Met Gly Pro Ile Ser
                85                  90                  95
Glu Val Asp Met Thr Tyr Ser Met Asp Cys Tyr Phe Arg Gln Ser Trp
            100                 105                 110
Val Asp Arg Arg Leu Ala Phe Gln Gly Gly Lys Glu Thr Leu Ala Leu
```

```
        115                 120                 125
Ser Ile Ser Met Leu Ala Arg Ile Trp Lys Pro Asp Thr Tyr Phe Tyr
    130                 135                 140
Asn Gly Lys His Ser Tyr Leu His Thr Ile Thr Ser Pro Asn Lys Phe
145                 150                 155                 160
Val Arg Leu Tyr Gln Asp Gly Arg Val Leu Tyr Ser Ser Arg Leu Thr
                165                 170                 175
Ile Lys Ala Gly Cys Pro Met Asn Leu Glu Asn Phe Pro Met Asp Thr
            180                 185                 190
Gln Arg Cys Pro Leu Gln Phe Gly Ser Phe Gly Tyr Thr Lys Arg Asp
        195                 200                 205
Val Ile Tyr Lys Trp Asn Ser Ala Arg Gln Val Ala Ile Ala Glu Asp
    210                 215                 220
Met Lys Leu Ser Gln Phe Asp Leu Val Ala Asn Pro Thr Ala Asn Tyr
225                 230                 235                 240
Ser Ala Ser Thr Thr Leu Ser His Ala Glu Tyr Ser Met Leu Leu Val
                245                 250                 255
Tyr Phe His Leu Gln Arg His Met Gly Asn Phe Leu Ile Gln Val Tyr
            260                 265                 270
Gly Pro Cys Val Leu Leu Val Val Leu Ser Trp Val Ser Phe Trp Leu
        275                 280                 285
Asn Arg Glu Ala Thr Ala Asp Arg Val Ser Leu Gly Ile Thr Thr Val
    290                 295                 300
Leu Thr Met Thr Phe Leu Gly Leu Glu Ala Arg Thr Asp Leu Pro Lys
305                 310                 315                 320
Val Pro Tyr Pro Thr Ala Leu Asp Phe Phe Val Phe Leu Ser Phe Ala
                325                 330                 335
Phe Ile Phe Ala Thr Ile Ile Gln Phe Ala Val Val His Tyr Phe Thr
            340                 345                 350
Lys Tyr Gly Ser Gly Glu Cys Tyr Phe Ser Ser Asp Leu Ser Glu Ser
        355                 360                 365
Glu Thr Ser Ser Asp Glu Glu Glu Ala Asp Thr Arg Pro Leu Lys Lys
    370                 375                 380
Glu Pro Ile Ser Gln Ser Asn Lys Arg Thr Ala Gly Asn Leu Ala Thr
385                 390                 395                 400
Arg Glu His Pro Ser Arg Asn Phe Thr Met Ser Glu Asp Gly Met Ile
                405                 410                 415
Glu Val Ile Pro Leu Ser Ala Ile Pro Glu Pro Ser Lys Asn Pro Ala
            420                 425                 430
Met Gly His Trp Gln Met Ser Cys Val Ala Cys Ser Pro Pro Pro Arg
        435                 440                 445
Gln Thr Pro Pro Ser Arg Lys Glu Ser Gly Arg Arg Arg Arg Arg Arg
    450                 455                 460
Thr Pro Arg Tyr Asn Ser Val Ser Lys Ile Asp Arg Ala Ser Arg Ile
465                 470                 475                 480
Val Phe Pro Leu Phe Phe Leu Ala Ile Asn Val Phe Tyr Trp Phe Ala
                485                 490                 495
Tyr Leu Ser Arg Ser Glu Arg Ile Asn Tyr Tyr Asn Val Asn Ser Asn
            500                 505                 510
Gly Thr
```

```
<210> 5
<211> 1798
<212> DNA
<213> Apis mellifera

<220>
<221> source
<222> 1..1798
<223> /mol_type="unassigned DNA"
      /note="ligand-gated ion channel homologue 3 subunit "
      /organism="Apis mellifera"
```

<400> 5

```
aaaagcatcc gagacttcgg tttggagaat atcctcgacg agcatcgtgt cgagattgga      60
cagtgctcat gaacgaatct gtccgtacgg tggtgaaaaa agtgtgaaaa aaagcccttt     120
taggagccag atcagtgccg ggaaggagag gcattgactc ctggttcaag acaagatctc     180
cttgacaatg tctgcggatc ctgtcgacga cttttccgcc aatgtggttc catcttttca     240
atgcaacgtt ttttggcaac tcgatatcga gatgcaaaca tctatcgaga tgacgtcctc     300
ttttatctgc tgacataatt ttgaagaatg catcacagga tgtggttgca gcagatcttt     360
atcctgctgc agatgattca tctaattgct tgggccagtc ttgaaaacac aggaatatct     420
gatagattgg aaaatgtgac gcaaacaata tcacgaatcc ttgatggtta cgatattcga     480
ttaaggccaa atttcggcgg agaacccctg ttagtcggga tggatcttac tatcgcgagc     540
ttcgatgcaa tctcggaagt aaacatggat tatacaataa caatgtattt aaatcaatat     600
tggaaagacg aaagattggc attttctcaa gaagaagaag ttctcactct tagtggagat     660
tttgcggaaa agatttgggt tccagatact ttttttgcca atgacaaaaa tagtttctta     720
cacgatgtga ccgagcgtaa taaactcgtt cggttgtccg gagacggatc tgtcacttat     780
ggcatgagat tcacgacgac cctggcctgc atgatggatc tgcactacta tccgctcgat     840
tcgcagaact gcaccgtaga aatcgagagc tacggataca cggtgttaga cgtggtaatg     900
tattggaaag agactccagt ccgtggtgtc gaggaagcgg aactgccaca attcacgata     960
attggttacg aaacgaatga tcgtaaagag aaactggcaa ctggtatata ccagagatta    1020
tcattgagtt tcaaacttca aaggaacatt ggatatttcg ttttccagac ttatttacct    1080
agtattttga ttgtaatgtt aagttgggtc agtttttgga taaatcatga agcaaccagc    1140
gccagagtag cactcggtat aacaacagtc cttacaatga ccacaatttc aacaggtgtc    1200
cgtagctcac tgccacgtat tagctacgtg aaagctatcg acatttactt agtaatgtgt    1260
ttcgtttttg tattcgccgc tcttttggaa tatgcagcgg ttaattatac ttattggggt    1320
gccagagcta aaaagaaatc gaaaaagaaa gaatctgatg ataaaaaagt gatttcctcg    1380
aagtccggaa gcaaagcaaa ttctcctttt cctggttcaa cggaagcgga tataatagag    1440
cttcaagatc tcaggatgtc tccattacca agtataagaa acagatcagg tctggttagt    1500
ggcagttcga cgcctggaac cggaagagaa catgatccgg ccaagtttcc ccctagcttt    1560
cgaatttcca gagtcgcggc ctacaatact tatgggagaa acgctggtct cagatacaga    1620
ggacctaaac aaaataagcc taaggtacta catgcaatac gaagaggagc atctgtattg    1680
cgtgtatcaa tgcctaaaat aaaagatgtg aatattatcg acaaatattc aagaattata    1740
tttccagtca gttttatgct gttcaacgcc atctattgga tattttatgt cctctgag      1798
```

<210> 6
<211> 489
<212> PRT
<213> Apis mellifera

<220>
<223> ligand-gated ion channel homologue 3

<400> 6
Met His His Arg Met Trp Leu Gln Gln Ile Phe Ile Leu Leu Gln Met
1               5                   10                  15
Ile His Leu Ile Ala Trp Ala Ser Leu Glu Asn Thr Gly Ile Ser Asp
            20                  25                  30
Arg Leu Glu Asn Val Thr Gln Thr Ile Ser Arg Ile Leu Asp Gly Tyr
        35                  40                  45
Asp Ile Arg Leu Arg Pro Asn Phe Gly Gly Glu Pro Leu Leu Val Gly
    50                  55                  60
Met Asp Leu Thr Ile Ala Ser Phe Asp Ala Ile Ser Glu Val Asn Met
65                  70                  75                  80
Asp Tyr Thr Ile Thr Met Tyr Leu Asn Gln Tyr Trp Lys Asp Glu Arg
                85                  90                  95
Leu Ala Phe Ser Gln Glu Glu Glu Val Leu Thr Leu Ser Gly Asp Phe
            100                 105                 110
Ala Glu Lys Ile Trp Val Pro Asp Thr Phe Phe Ala Asn Asp Lys Asn
        115                 120                 125
Ser Phe Leu His Asp Val Thr Glu Arg Asn Lys Leu Val Arg Leu Ser
    130                 135                 140
Gly Asp Gly Ser Val Thr Tyr Gly Met Arg Phe Thr Thr Thr Leu Ala
145                 150                 155                 160
Cys Met Met Asp Leu His Tyr Tyr Pro Leu Asp Ser Gln Asn Cys Thr
                165                 170                 175
Val Glu Ile Glu Ser Tyr Gly Tyr Thr Val Leu Asp Val Val Met Tyr
            180                 185                 190
Trp Lys Glu Thr Pro Val Arg Gly Val Glu Glu Ala Glu Leu Pro Gln
        195                 200                 205
Phe Thr Ile Ile Gly Tyr Glu Thr Asn Asp Arg Lys Glu Lys Leu Ala
    210                 215                 220
Thr Gly Ile Tyr Gln Arg Leu Ser Leu Ser Phe Lys Leu Gln Arg Asn
225                 230                 235                 240
Ile Gly Tyr Phe Val Phe Gln Thr Tyr Leu Pro Ser Ile Leu Ile Val
                245                 250                 255
Met Leu Ser Trp Val Ser Phe Trp Ile Asn His Glu Ala Thr Ser Ala
            260                 265                 270
Arg Val Ala Leu Gly Ile Thr Thr Val Leu Thr Met Thr Thr Ile Ser
        275                 280                 285
Thr Gly Val Arg Ser Ser Leu Pro Arg Ile Ser Tyr Val Lys Ala Ile
    290                 295                 300
Asp Ile Tyr Leu Val Met Cys Phe Val Phe Val Phe Ala Ala Leu Leu
305                 310                 315                 320
Glu Tyr Ala Ala Val Asn Tyr Thr Tyr Trp Gly Ala Arg Ala Lys Lys
                325                 330                 335
Lys Ser Lys Lys Lys Glu Ser Asp Asp Lys Lys Val Ile Ser Ser Lys
            340                 345                 350
Ser Gly Ser Lys Ala Asn Ser Pro Phe Pro Gly Ser Thr Glu Ala Asp
        355                 360                 365
Ile Ile Glu Leu Gln Asp Leu Arg Met Ser Pro Leu Pro Ser Ile Arg
    370                 375                 380
Asn Arg Ser Gly Leu Val Ser Gly Ser Ser Thr Pro Gly Thr Gly Arg
385                 390                 395                 400
Glu His Asp Pro Ala Lys Phe Pro Pro Ser Phe Arg Ile Ser Arg Val
                405                 410                 415
Ala Ala Tyr Asn Thr Tyr Gly Arg Asn Ala Gly Leu Arg Tyr Arg Gly
            420                 425                 430

```
Pro Lys Gln Asn Lys Pro Lys Val Leu His Ala Ile Arg Arg Gly Ala
        435                 440                 445
Ser Val Leu Arg Val Ser Met Pro Lys Ile Lys Asp Val Asn Ile Ile
    450                 455                 460
Asp Lys Tyr Ser Arg Ile Ile Phe Pro Val Ser Phe Met Leu Phe Asn
465                 470                 475                 480
Ala Ile Tyr Trp Ile Phe Tyr Val Leu
                485
```

```
<210> 7
<211> 65
<212> DNA
<213> Artificial Sequence

<220>
<221> source
<222> 1..65
<223> /mol_type="unassigned DNA"
      /note="amorce PL014"
      /organism="Artificial Sequence"

<400> 7
ctagagcggc cgcgagctca gcgatctaga gaaagcttga gttaacgagc tagcgaggta     60

ccgac                                                                 65


<210> 8
<211> 65
<212> DNA
<213> Artificial Sequence

<220>
<221> source
<222> 1..65
<223> /mol_type="unassigned DNA"
      /note="amorce PL015"
      /organism="Artificial Sequence"

<400> 8
ttaagtcggt acctcgctag ctcgttaact caagctttct ctagatcgct gagctcgcgg     60

ccgct                                                                 65


<210> 9
<211> 22
<212> DNA
<213> Artificial Sequence

<220>
<221> source
<222> 1..22
<223> /mol_type="unassigned DNA"
      /note="amgrd-005AS"
      /organism="Artificial Sequence"

<400> 9
cacgatctgc agtggcttct cg                                              22


<210> 10
<211> 24
```

<212> DNA
<213> Artificial Sequence

<220>
<221> source
<222> 1..24
<223> /mol_type="unassigned DNA"
/note="amgrd-004AS"
/organism="Artificial Sequence"

<400> 10
ccagaaggaa acccaagaca ggac 24

<210> 11
<211> 28
<212> DNA
<213> Artificial Sequence

<220>
<221> source
<222> 1..28
<223> /mol_type="unassigned DNA"
/note="amgrd-001S"
/organism="Artificial Sequence"

<400> 11
gaattgatga atgaatcagg acgtattg 28

<210> 12
<211> 23
<212> DNA
<213> Artificial Sequence

<220>
<221> source
<222> 1..23
<223> /mol_type="unassigned DNA"
/note="amgrd-002AS"
/organism="Artificial Sequence"

<400> 12
cgaccagtct cacgtgccat ttg 23

<210> 13
<211> 25
<212> DNA
<213> Artificial Sequence

<220>
<221> source
<222> 1..25
<223> /mol_type="unassigned DNA"
/note="amlcch3-004AS"
/organism="Artificial Sequence"

<400> 13
gaactgccac taaccagacc tgatc 25

<210> 14

<211> 26
<212> DNA
<213> Artificial Sequence

<220>
<221> source
<222> 1..26
<223> /mol_type="unassigned DNA"
/note="amlcch3-003AS"
/organism="Artificial Sequence"

<400> 14
ggtaatggag acatcctgag atcttg 26

<210> 15
<211> 25
<212> DNA
<213> Artificial Sequence

<220>
<221> source
<222> 1..25
<223> /mol_type="unassigned DNA"
/note="amlcch3-001S"
/organism="Artificial Sequence"

<400> 15
catgcatcac aggatgtggt tgcag 25

<210> 16
<211> 31
<212> DNA
<213> Artificial Sequence

<220>
<221> source
<222> 1..31
<223> /mol_type="unassigned DNA"
/note="amlcch3-002AS"
/organism="Artificial Sequence"

<400> 16
ctcagaggac ataaaatatc caatagatgg c 31

<210> 17
<211> 25
<212> DNA
<213> Artificial Sequence

<220>
<221> source
<222> 1..25
<223> /mol_type="unassigned DNA"
/note="amrdl-004AS"
/organism="Artificial Sequence"

<400> 17
cttgacgtag gagattttcg gtaac 25

```
<210> 18
<211> 25
<212> DNA
<213> Artificial Sequence

<220>
<221> source
<222> 1..25
<223> /mol_type="unassigned DNA"
      /note="amrdl-003AS"
      /organism="Artificial Sequence"

<400> 18
gttcgtcgac gacattaggg tggtc                                          25


<210> 19
<211> 26
<212> DNA
<213> Artificial Sequence

<220>
<221> source
<222> 1..26
<223> /mol_type="unassigned DNA"
      /note="amrdl-001S"
      /organism="Artificial Sequence"

<400> 19
catgctgaat gacgtcaaca tctccg                                         26


<210> 20
<211> 24
<212> DNA
<213> Artificial Sequence

<220>
<221> source
<222> 1..24
<223> /mol_type="unassigned DNA"
      /note="amrdl-002AS"
      /organism="Artificial Sequence"

<400> 20
ctcttcgctt atttcgcctc ctcg                                           24
```